(19) Europäisches Patentamt
European Patent Office
Office européen des brevets

(11) **EP 2 686 338 B1**

(12) **EUROPEAN PATENT SPECIFICATION**

(45) Date of publication and mention of the grant of the patent:
**28.11.2018 Bulletin 2018/48**

(51) Int Cl.:
***C07K 14/00*** *(2006.01)*    ***A61L 27/22*** *(2006.01)*

(21) Application number: **12713898.0**

(22) Date of filing: **14.03.2012**

(86) International application number:
**PCT/EP2012/054458**

(87) International publication number:
**WO 2012/123495 (20.09.2012 Gazette 2012/38)**

(54) **FUNCTIONALIZED BIOMATERIALS FOR TISSUE REGENERATION**

FUNKTIONALISIERTE BIOMATERIALIEN ZUR GEWEBEREGENERATION

BIOMATÉRIAUX FONCTIONNALISÉS DESTINÉS À LA RÉGÉNÉRATION TISSULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2011 PCT/EP2011/053838**

(43) Date of publication of application:
**22.01.2014 Bulletin 2014/04**

(73) Proprietor: **Nanomed3d S.r.l.**
**20129 Milano (IT)**

(72) Inventors:
• **GELAIN, Fabrizio**
**I-20094 Corsico (IT)**
• **VESCOVI, Angelo Luigi**
**CH-6817 Maroggia (CH)**

(74) Representative: **Zaccaro, Elisabetta et al**
**Notarbartolo & Gervasi S.p.A.**
**Corso di Porta Vittoria, 9**
**20122 Milano (IT)**

(56) References cited:
• FABRIZIO GELAIN ET AL: "Transplantation of Nanostructured Composite Scaffolds Results in the Regeneration of Chronically Injured Spinal Cords", ACS NANO, vol. 5, no. 1, 25 January 2011 (2011-01-25), pages 227-236, XP055010204, ISSN: 1936-0851, DOI: 10.1021/nn102461w

• GELAIN FABRIZIO ET AL: "Designer self-assembling Peptide nanofiber scaffolds for adult mouse neural stem cell 3-dimensional cultures", PLOS ONE, PUBLIC LIBRARY OF SCIENCE, UNITED STATES, vol. 1, no. 1, 27 December 2006 (2006-12-27), pages E119-1, XP002489970, ISSN: 1932-6203, DOI: 10.1371/JOURNAL.PONE.0000119

• FABRIZIO GELAIN ET AL: "BMHP1-derived self-assembling peptides: hierarchically assembled structures with self-healing propensity and potential for tissue engineering applications", ACS NANO, AMERICAN CHEMICAL SOCIETY, US, vol. 5, no. 3, 11 February 2011 (2011-02-11), pages 1845-1859, XP002658201, ISSN: 1936-086X, DOI: 10.1021/NN102663A [retrieved on 2011-02-11]

• DATABASE UniProt [Online] 1 May 2007 (2007-05-01), "SubName: Full=Putative uncharacterized protein;", XP002661856, retrieved from EBI accession no. UNIPROT:A4HUY3 Database accession no. A4HUY3

• KISIDAY J ET AL: "Self-assembling peptide hydrogel fosters chondrocyte extracellular matrix production and cell division: Implications for cartilage tissue repair", PROCEEDINGS OF THE NATIONAL ACADEMY OF SCIENCES OF USA, NATIONAL ACADEMY OF SCIENCE, WASHINGTON, DC; US, vol. 99, no. 15, 23 July 2002 (2002-07-23) , pages 9996-10001, XP002278385, ISSN: 0027-8424, DOI: 10.1073/PNAS.142309999

EP 2 686 338 B1

- **GRZEGORZ S. NOWAKOWSKI ET AL: "A Specific Heptapeptide from a Phage Display Peptide Library Homes to Bone Marrow and Binds to Primitive Hematopoietic Stem Cells", STEM CELLS, vol. 22, no. 6, 1 November 2004 (2004-11-01), pages 1030-1038, XP055010208, ISSN: 1066-5099, DOI: 10.1634/stemcells.22-6-1030**

- **F. GELAIN ET AL: "New bioactive motifs and their use in functionalized self-assembling peptides for NSC differentiation and neural tissue engineering", NANOSCALE, vol. 4, no. 9, 24 February 2012 (2012-02-24), pages 2946-2957, XP055039091, ISSN: 2040-3364, DOI: 10.1039/c2nr30220a**

SEQUENCE LISTING

<110> NANOMED3D S.r.l.

<120> FUNCTIONALIZED BIOMATERIALS FOR TISSUE REGENERATION

<130> 10933PTWO

<150> PCT/EP2011/053838
<151> 2011-03-15

<160> 75

<170> BiSSAP 1.0

<210> 1
<211> 7
<212> PRT
<213> Artificial Sequence

<220>
<221> SOURCE
<222> 1..7
<223> /mol_type="protein"
/note="Prepared by synthesis"
/organism="Artificial Sequence"

<400> 1

```
                              Phe Ala Gln Arg Val Pro Pro
                              1               5
```

<210> 2
<211> 7
<212> PRT
<213> Artificial Sequence

<220>
<221> SOURCE
<222> 1..7
<223> /mol_type="protein"
/note="Prepared by synthesis"
/organism="Artificial Sequence"

<400> 2

```
                              Gln His Leu Pro Arg Asp His
                              1               5
```

<210> 3
<211> 7
<212> PRT
<213> Artificial Sequence

<220>
<221> SOURCE

<222> 1..7
<223> /mol_type="protein"
/note="Prepared by synthesis"
/organism="Artificial Sequence"

<400> 3

```
Ser Ser Leu Ser Val Asn Asp
1               5
```

<210> 4
<211> 7
<212> PRT
<213> Artificial Sequence

<220>
<221> SOURCE
<222> 1..7
<223> /mol_type="protein"
/note="Prepared by synthesis"
/organism="Artificial Sequence"

<400> 4

```
Tyr Ile Ile Pro Met His Asp
1               5
```

<210> 5
<211> 7
<212> PRT
<213> Artificial Sequence

<220>
<221> SOURCE
<222> 1..7
<223> /mol_type="protein"
/note="Prepared by synthesis"
/organism="Artificial Sequence"

<400> 5

```
Ser Leu Pro Lys Leu Pro Pro
1               5
```

<210> 6
<211> 7
<212> PRT
<213> Artificial Sequence

<220>
<221> SOURCE
<222> 1..7
<223> /mol_type="protein"
/note="Prepared by synthesis"
/organism="Artificial Sequence"

<400> 6

```
Thr Pro Leu Ser Ser His Ser
1               5
```

<210> 7
<211> 7
<212> PRT
<213> Artificial Sequence

<220>
<221> SOURCE
<222> 1..7
<223> /mol_type="protein"
/note="Prepared by synthesis"
/organism="Artificial Sequence"

<400> 7

```
Ser Ala Ser His Trp Gln Ile
1               5
```

<210> 8
<211> 7
<212> PRT
<213> Artificial Sequence

<220>
<221> SOURCE
<222> 1..7
<223> /mol_type="protein"
/note="Prepared by synthesis"
/organism="Artificial Sequence"

<400> 8

```
Leu Gln Ala Ile Pro Arg Asn
1               5
```

<210> 9
<211> 7
<212> PRT
<213> Artificial Sequence

<220>
<221> SOURCE
<222> 1..7
<223> /mol_type="protein"
/note="Prepared by synthesis"
/organism="Artificial Sequence"

<400> 9

```
Tyr Arg Met Pro Ile Trp Pro
1               5
```

<210> 10
<211> 7
<212> PRT
<213> Artificial Sequence

```
<220>
<221> SOURCE
<222> 1..7
<223> /mol_type="protein"
/note="Prepared by synthesis"
/organism="Artificial Sequence"

<400> 10
```

Lys Leu Pro Gly Trp Ser Gly
1                   5

```
<210> 11
<211> 7
<212> PRT
<213> Artificial Sequence

<220>
<221> SOURCE
<222> 1..7
<223> /mol_type="protein"
/note="Prepared by synthesis"
/organism="Artificial Sequence"

<400> 11
```

His Ala Ile Tyr Pro Arg His

    1                   5

```
<210> 12
<211> 7
<212> PRT
<213> Artificial Sequence

<220>
<221> SOURCE
<222> 1..7
<223> /mol_type="protein"
/note="Prepared by synthesis"
/organism="Artificial Sequence"

<400> 12
```

Gly Glu Thr Arg Ala Pro Leu
1                   5

```
<210> 13
<211> 7
<212> PRT
<213> Artificial Sequence

<220>
<221> SOURCE
<222> 1..7
<223> /mol_type="protein"
```

/note="Prepared by synthesis"
/organism="Artificial Sequence"

<400> 13

```
Ala Leu Thr Pro Trp Ala Phe
1               5
```

<210> 14
<211> 7
<212> PRT
<213> Artificial Sequence

<220>
<221> SOURCE
<222> 1..7
<223> /mol_type="protein"
/note="Prepared by synthesis"
/organism="Artificial Sequence"

<400> 14

```
Gly Lys Pro Met Pro Pro Met
1               5
```

<210> 15
<211> 7
<212> PRT
<213> Artificial Sequence

<220>
<221> SOURCE
<222> 1..7
<223> /mol_type="protein"
/note="Prepared by synthesis"
/organism="Artificial Sequence"

<400> 15

```
Ser Ile Leu Pro Tyr Pro Tyr
1               5
```

<210> 16
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 22..22
<223> AMIDATION

<400> 16

```
Phe Ala Gln Arg Val Pro Pro Gly Gly Gly Leu Asp Leu Lys Leu Asp
1               5                   10                  15
Leu Lys Leu Asp Leu Lys
            20
```

<210> 17
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<220>
<221> MOD_RES
<222> 22..22
<223> AMIDATION

<400> 17

```
Phe Ala Gln Arg Val Pro Pro Gly Gly Gly Leu Asp Leu Lys Leu Asp
1               5                   10                  15
Leu Lys Leu Asp Leu Lys
            20
```

<210> 18
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 22..22
<223> AMIDATION

<400> 18

```
Gln His Leu Pro Arg Asp His Gly Gly Gly Leu Asp Leu Lys Leu Asp
1               5                   10                  15
Leu Lys Leu Asp Leu Lys
            20
```

<210> 19
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<220>
<221> MOD_RES
<222> 22..22
<223> AMIDATION

<400> 19

```
Gln His Leu Pro Arg Asp His Gly Gly Gly Leu Asp Leu Lys Leu Asp
1               5                   10                  15
Leu Lys Leu Asp Leu Lys
            20
```

<210> 20
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 22..22
<223> AMIDATION

<400> 20

```
Ser Ser Leu Ser Val Asn Asp Gly Gly Gly Leu Asp Leu Lys Leu Asp
1               5                   10                  15
Leu Lys Leu Asp Leu Lys
```

20

```
<210> 21
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<220>
<221> MOD_RES
<222> 22..22
<223> AMIDATION

<400> 21


        Ser Ser Leu Ser Val Asn Asp Gly Gly Gly Leu Asp Leu Lys Leu Asp
        1               5                   10                  15
        Leu Lys Leu Asp Leu Lys
                    20


<210> 22
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 22..22
<223> AMIDATION

<400> 22


        Tyr Ile Ile Pro Met His Asp Gly Gly Gly Leu Asp Leu Lys Leu Asp
        1               5                   10                  15
        Leu Lys Leu Asp Leu Lys
                    20


<210> 23
<211> 22
```

<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<220>
<221> MOD_RES
<222> 22..22
<223> AMIDATION

<400> 23

```
Tyr Ile Ile Pro Met His Asp Gly Gly Gly Leu Asp Leu Lys Leu Asp
1               5                   10                  15
Leu Lys Leu Asp Leu Lys
            20
```

<210> 24
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 22..22
<223> AMIDATION

<400> 24

```
Ser Leu Pro Lys Leu Pro Pro Gly Gly Gly Leu Asp Leu Lys Leu Asp
1               5                   10                  15
Leu Lys Leu Asp Leu Lys
            20
```

<210> 25
<211> 22
<212> PRT
<213> Artificial Sequence

<220>
<221> SOURCE

<222> 1..22
<223> /mol_type="protein"
/note="Prepared by synthesis"
/organism="Artificial Sequence"

<220>
<221> MOD_RES
<222> 1..1
<223> ACETYLATION

<220>
<221> MOD_RES
<222> 22..22
<223> AMIDATION

<400> 25

```
    Ser Leu Pro Lys Leu Pro Pro Gly Gly Gly Leu Asp Leu Lys Leu Asp
    1               5                   10                  15
    Leu Lys Leu Asp Leu Lys
                    20
```

<210> 26
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 22..22
<223> AMIDATION

<400> 26

```
    Thr Pro Leu Ser Ser His Ser Gly Gly Gly Leu Asp Leu Lys Leu Asp
    1               5                   10                  15
    Leu Lys Leu Asp Leu Lys
                    20
```

<210> 27
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<220>
<221> MOD_RES
<222> 22..22
<223> AMIDATION

<400> 27

```
Thr Pro Leu Ser Ser His Ser Gly Gly Gly Leu Asp Leu Lys Leu Asp
1               5                   10                  15
Leu Lys Leu Asp Leu Lys
            20
```

<210> 28
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 22..22
<223> AMIDATION

<400> 28

```
Ser Ala Ser His Trp Gln Ile Gly Gly Gly Leu Asp Leu Lys Leu Asp
1               5                   10                  15
Leu Lys Leu Asp Leu Lys
            20
```

<210> 29
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<220>
<221> MOD_RES
<222> 22..22
<223> AMIDATION

<400> 29

```
        Ser Ala Ser His Trp Gln Ile Gly Gly Gly Leu Asp Leu Lys Leu Asp
        1               5                   10                  15
        Leu Lys Leu Asp Leu Lys
                        20
```

<210> 30
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 22..22
<223> AMIDATION

<400> 30

```
        Leu Gln Ala Ile Pro Arg Asn Gly Gly Gly Leu Asp Leu Lys Leu Asp
        1               5                   10                  15
        Leu Lys Leu Asp Leu Lys
                        20
```

<210> 31
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<220>
<221> MOD_RES
<222> 22..22
<223> AMIDATION

<400> 31

```
Leu Gln Ala Ile Pro Arg Asn Gly Gly Gly Leu Asp Leu Lys Leu Asp
1               5                   10                  15
Leu Lys Leu Asp Leu Lys
            20
```

<210> 32
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 22..22
<223> AMIDATION

<400> 32

```
Tyr Arg Met Pro Ile Trp Pro Gly Gly Gly Leu Asp Leu Lys Leu Asp
1               5                   10                  15
Leu Lys Leu Asp Leu Lys
            20
```

<210> 33
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<220>
<221> MOD_RES
<222> 22..22
<223> AMIDATION

<400> 33

```
Tyr Arg Met Pro Ile Trp Pro Gly Gly Gly Leu Asp Leu Lys Leu Asp
1               5                   10                  15
Leu Lys Leu Asp Leu Lys
                20
```

<210> 34
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 22..22
<223> AMIDATION

<400> 34

```
Lys Leu Pro Gly Trp Ser Gly Gly Gly Gly Leu Asp Leu Lys Leu Asp
1               5                   10                  15
Leu Lys Leu Asp Leu Lys
                20
```

<210> 35
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<220>
<221> MOD_RES
<222> 22..22
<223> AMIDATION

<400> 35

```
Lys Leu Pro Gly Trp Ser Gly Gly Gly Gly Leu Asp Leu Lys Leu Asp
1               5                   10                  15
Leu Lys Leu Asp Leu Lys
                20
```

<210> 36
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 22..22
<223> AMIDATION

<400> 36

```
His Ala Ile Tyr Pro Arg His Gly Gly Gly Leu Asp Leu Lys Leu Asp
1               5                   10                  15
Leu Lys Leu Asp Leu Lys
            20
```

<210> 37
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<220>
<221> MOD_RES
<222> 22..22
<223> AMIDATION

<400> 37

```
His Ala Ile Tyr Pro Arg His Gly Gly Gly Leu Asp Leu Lys Leu Asp
1               5                   10                  15
Leu Lys Leu Asp Leu Lys
            20
```

<210> 38
<211> 22
<212> PRT
<213> Artificial Sequence

```
<220>
<221> SOURCE
<222> 1..22
<223> /mol_type="protein"
/note="Prepared by synthesis"
/organism="Artificial Sequence"


<220>
<221> MOD_RES
<222> 22..22
<223> AMIDATION


<400> 38

        Gly Glu Thr Arg Ala Pro Leu Gly Gly Gly Leu Asp Leu Lys Leu Asp
        1                   5                   10                  15
        Leu Lys Leu Asp Leu Lys
                        20

<210> 39
<211> 22
<212> PRT
<213> Artificial Sequence

<220>
<221> SOURCE
<222> 1..22
<223> /mol_type="protein"
/note="Prepared by synthesis"
/organism="Artificial Sequence"


<220>
<221> MOD_RES
<222> 1..1
<223> ACETYLATION


<220>
<221> MOD_RES
<222> 22..22
<223> AMIDATION


<400> 39

        Gly Glu Thr Arg Ala Pro Leu Gly Gly Gly Leu Asp Leu Lys Leu Asp
        1                   5                   10                  15
        Leu Lys Leu Asp Leu Lys
                        20

<210> 40
<211> 22
<212> PRT
<213> Artificial Sequence

<220>
<221> SOURCE
<222> 1..22
<223> /mol_type="protein"
/note="Prepared by synthesis"
```

/organism="Artificial Sequence"

```
<220>
<221> MOD_RES
<222> 22..22
<223> AMIDATION
```

```
<400> 40
```

```
Ala Leu Thr Pro Trp Ala Phe Gly Gly Gly Leu Asp Leu Lys Leu Asp
1               5                   10                  15
Leu Lys Leu Asp Leu Lys
            20
```

```
<210> 41
<211> 22
<212> PRT
<213> Artificial Sequence
```

```
<220>
<221> SOURCE
<222> 1..22
<223> /mol_type="protein"
/note="Prepared by synthesis"
/organism="Artificial Sequence"
```

```
<220>
<221> MOD_RES
<222> 1..1
<223> ACETYLATION
```

```
<220>
<221> MOD_RES
<222> 22..22
<223> AMIDATION
```

```
<400> 41
```

```
Ala Leu Thr Pro Trp Ala Phe Gly Gly Gly Leu Asp Leu Lys Leu Asp
1               5                   10                  15
Leu Lys Leu Asp Leu Lys
            20
```

```
<210> 42
<211> 22
<212> PRT
<213> Artificial Sequence
```

```
<220>
<221> SOURCE
<222> 1..22
<223> /mol_type="protein"
/note="Prepared by synthesis"
/organism="Artificial Sequence"
```

```
<220>
<221> MOD_RES
<222> 22..22
```

<223> AMIDATION

<400> 42

```
Gly Lys Pro Met Pro Pro Met Gly Gly Gly Leu Asp Leu Lys Leu Asp
1               5                   10                  15
Leu Lys Leu Asp Leu Lys
            20
```

<210> 43
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<220>
<221> MOD_RES
<222> 22..22
<223> AMIDATION

<400> 43

```
Gly Lys Pro Met Pro Pro Met Gly Gly Gly Leu Asp Leu Lys Leu Asp
1               5                   10                  15
Leu Lys Leu Asp Leu Lys
            20
```

<210> 44
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 22..22
<223> AMIDATION

<400> 44

```
        Ser Ile Leu Pro Tyr Pro Tyr Gly Gly Gly Leu Asp Leu Lys Leu Asp
        1               5                   10                  15
        Leu Lys Leu Asp Leu Lys
                    20
```

<210> 45
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<220>
<221> MOD_RES
<222> 22..22
<223> AMIDATION

<400> 45

```
        Ser Ile Leu Pro Tyr Pro Tyr Gly Gly Gly Leu Asp Leu Lys Leu Asp
        1               5                   10                  15
        Leu Lys Leu Asp Leu Lys
                    20
```

<210> 46
<211> 22
<212> PRT
<213> Artificial Sequence

<220>
<221> SOURCE
<222> 1..22
<223> /mol_type="protein"
/note="Prepared by synthesis"
/organism="Artificial Sequence"

<400> 46

```
        Phe Ala Gln Arg Val Pro Pro Gly Gly Gly Leu Asp Leu Lys Leu Asp
        1               5                   10                  15
        Leu Lys Leu Asp Leu Lys
                    20
```

<210> 47
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<400> 47

```
Phe Ala Gln Arg Val Pro Pro Gly Gly Gly Leu Asp Leu Lys Leu Asp
1               5                   10                  15
Leu Lys Leu Asp Leu Lys
            20
```

<210> 48
<211> 22
<212> PRT
<213> Artificial Sequence

<220>
<221> SOURCE
<222> 1..22
<223> /mol_type="protein"
/note="Prepared by synthesis"
/organism="Artificial Sequence"

<400> 48

```
Gln His Leu Pro Arg Asp His Gly Gly Gly Leu Asp Leu Lys Leu Asp
1               5                   10                  15
Leu Lys Leu Asp Leu Lys
            20
```

<210> 49
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<400> 49

```
Gln His Leu Pro Arg Asp His Gly Gly Gly Leu Asp Leu Lys Leu Asp
1               5               10              15
Leu Lys Leu Asp Leu Lys
            20
```

<210> 50
<211> 22
<212> PRT
<213> Artificial Sequence

<220>
<221> SOURCE
<222> 1..22
<223> /mol_type="protein"
/note="Prepared by synthesis"
/organism="Artificial Sequence"

<400> 50

```
Ser Ser Leu Ser Val Asn Asp Gly Gly Gly Leu Asp Leu Lys Leu Asp
1               5               10              15
Leu Lys Leu Asp Leu Lys
            20
```

<210> 51
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<400> 51

```
Ser Ser Leu Ser Val Asn Asp Gly Gly Gly Leu Asp Leu Lys Leu Asp
1               5               10              15
Leu Lys Leu Asp Leu Lys
            20
```

<210> 52
<211> 22
<212> PRT
<213> Artificial Sequence

<220>
<221> SOURCE
<222> 1..22
<223> /mol_type="protein"
/note="Prepared by synthesis"

/organism="Artificial Sequence"

<400> 52

```
        Tyr Ile Ile Pro Met His Asp Gly Gly Gly Leu Asp Leu Lys Leu Asp
        1               5                   10                  15
        Leu Lys Leu Asp Leu Lys
                    20
```

<210> 53
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<400> 53

```
        Tyr Ile Ile Pro Met His Asp Gly Gly Gly Leu Asp Leu Lys Leu Asp
        1               5                   10                  15
        Leu Lys Leu Asp Leu Lys
                    20
```

<210> 54
<211> 22
<212> PRT
<213> Artificial Sequence

<220>
<221> SOURCE
<222> 1..22
<223> /mol_type="protein"
/note="Prepared by synthesis"
/organism="Artificial Sequence"

<400> 54

```
        Ser Leu Pro Lys Leu Pro Pro Gly Gly Gly Leu Asp Leu Lys Leu Asp
        1               5                   10                  15
        Leu Lys Leu Asp Leu Lys
                    20
```

<210> 55
<211> 22
<212> PRT
<213> Artificial Sequence

<220>

<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<400> 55

```
Ser Leu Pro Lys Leu Pro Pro Gly Gly Gly Leu Asp Leu Lys Leu Asp
1               5               10              15
Leu Lys Leu Asp Leu Lys
            20
```

<210> 56
<211> 22
<212> PRT
<213> Artificial Sequence

<220>
<221> SOURCE
<222> 1..22
<223> /mol_type="protein"
/note="Prepared by synthesis"
/organism="Artificial Sequence"

<400> 56

```
Thr Pro Leu Ser Ser His Ser Gly Gly Gly Leu Asp Leu Lys Leu Asp
1               5               10              15
Leu Lys Leu Asp Leu Lys
            20
```

<210> 57
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<400> 57

39

```
Thr Pro Leu Ser Ser His Ser Gly Gly Gly Leu Asp Leu Lys Leu Asp
1                   5                   10                  15
Leu Lys Leu Asp Leu Lys
            20
```

<210> 58
<211> 22
<212> PRT
<213> Artificial Sequence

<220>
<221> SOURCE
<222> 1..22
<223> /mol_type="protein"
/note="Prepared by synthesis"
/organism="Artificial Sequence"

<400> 58

```
Ser Ala Ser His Trp Gln Ile Gly Gly Gly Leu Asp Leu Lys Leu Asp
1                   5                   10                  15
Leu Lys Leu Asp Leu Lys
            20
```

<210> 59
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<400> 59

```
Ser Ala Ser His Trp Gln Ile Gly Gly Gly Leu Asp Leu Lys Leu Asp
1                   5                   10                  15
Leu Lys Leu Asp Leu Lys
            20
```

<210> 60
<211> 22
<212> PRT
<213> Artificial Sequence

<220>
<221> SOURCE
<222> 1..22
<223> /mol_type="protein"
/note="Prepared by synthesis"

/organism="Artificial Sequence"

<400> 60

```
Leu Gln Ala Ile Pro Arg Asn Gly Gly Gly Leu Asp Leu Lys Leu Asp
1               5                   10                  15
Leu Lys Leu Asp Leu Lys
            20
```

<210> 61
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<400> 61

```
Leu Gln Ala Ile Pro Arg Asn Gly Gly Gly Leu Asp Leu Lys Leu Asp
1               5                   10                  15
Leu Lys Leu Asp Leu Lys
            20
```

<210> 62
<211> 22
<212> PRT
<213> Artificial Sequence

<220>
<221> SOURCE
<222> 1..22
<223> /mol_type="protein"
/note="Prepared by synthesis"
/organism="Artificial Sequence"

<400> 62

```
Tyr Arg Met Pro Ile Trp Pro Gly Gly Gly Leu Asp Leu Lys Leu Asp
1               5                   10                  15
Leu Lys Leu Asp Leu Lys
            20
```

<210> 63
<211> 22
<212> PRT

<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<400> 63

```
Tyr Arg Met Pro Ile Trp Pro Gly Gly Gly Leu Asp Leu Lys Leu Asp
1               5                   10                  15

                    Leu Lys Leu Asp Leu Lys
                            20
```

<210> 64
<211> 22
<212> PRT
<213> Artificial Sequence

<220>
<221> SOURCE
<222> 1..22
<223> /mol_type="protein"
/note="Prepared by synthesis"
/organism="Artificial Sequence"

<400> 64

```
Lys Leu Pro Gly Trp Ser Gly Gly Gly Gly Leu Asp Leu Lys Leu Asp
1               5                   10                  15
Leu Lys Leu Asp Leu Lys
        20
```

<210> 65
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<400> 65

```
Lys Leu Pro Gly Trp Ser Gly Gly Gly Gly Leu Asp Leu Lys Leu Asp
1               5                   10                  15
Leu Lys Leu Asp Leu Lys
            20
```

<210> 66
<211> 22
<212> PRT
<213> Artificial Sequence

<220>
<221> SOURCE
<222> 1..22
<223> /mol_type="protein"
/note="Prepared by synthesis"
/organism="Artificial Sequence"

<400> 66

```
His Ala Ile Tyr Pro Arg His Gly Gly Gly Leu Asp Leu Lys Leu Asp
1               5                   10                  15
Leu Lys Leu Asp Leu Lys
            20
```

<210> 67
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<400> 67

```
His Ala Ile Tyr Pro Arg His Gly Gly Gly Leu Asp Leu Lys Leu Asp
1               5                   10                  15
Leu Lys Leu Asp Leu Lys
            20
```

<210> 68
<211> 22
<212> PRT
<213> Artificial Sequence

<220>
<221> SOURCE
<222> 1..22

<223> /mol_type="protein"
/note="Prepared by synthesis"
/organism="Artificial Sequence"

<400> 68

```
Gly Glu Thr Arg Ala Pro Leu Gly Gly Gly Leu Asp Leu Lys Leu Asp
1               5               10              15
Leu Lys Leu Asp Leu Lys
            20
```

<210> 69
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<400> 69

```
Gly Glu Thr Arg Ala Pro Leu Gly Gly Gly Leu Asp Leu Lys Leu Asp
1               5               10              15
Leu Lys Leu Asp Leu Lys
            20
```

<210> 70
<211> 22
<212> PRT
<213> Artificial Sequence

<220>
<221> SOURCE
<222> 1..22
<223> /mol_type="protein"
/note="Prepared by synthesis"
/organism="Artificial Sequence"

<400> 70

```
Ala Leu Thr Pro Trp Ala Phe Gly Gly Gly Leu Asp Leu Lys Leu Asp
1               5               10              15
Leu Lys Leu Asp Leu Lys
            20
```

<210> 71
<211> 22
<212> PRT
<213> Artificial Sequence

```
<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<400> 71

        Ala Leu Thr Pro Trp Ala Phe Gly Gly Gly Leu Asp Leu Lys Leu Asp
        1               5                   10                  15
        Leu Lys Leu Asp Leu Lys
                        20

<210> 72
<211> 22
<212> PRT
<213> Artificial Sequence

<220>
<221> SOURCE
<222> 1..22
<223> /mol_type="protein"
/note="Prepared by synthesis"
/organism="Artificial Sequence"

<400> 72

        Gly Lys Pro Met Pro Pro Met Gly Gly Gly Leu Asp Leu Lys Leu Asp
        1               5                   10                  15
        Leu Lys Leu Asp Leu Lys
                        20

<210> 73
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<400> 73
```

```
Gly Lys Pro Met Pro Pro Met Gly Gly Gly Leu Asp Leu Lys Leu Asp
1               5                   10                  15
Leu Lys Leu Asp Leu Lys
            20
```

<210> 74
<211> 22
<212> PRT
<213> Artificial Sequence

<220>
<221> SOURCE
<222> 1..22
<223> /mol_type="protein"
/note="Prepared by synthesis"
/organism="Artificial Sequence"

<400> 74

```
Ser Ile Leu Pro Tyr Pro Tyr Gly Gly Gly Leu Asp Leu Lys Leu Asp
1               5                   10                  15
Leu Lys Leu Asp Leu Lys
            20
```

<210> 75
<211> 22
<212> PRT
<213> Artificial Sequence

<220>
<221> SOURCE
<222> 1..22
<223> /mol_type="protein"
/note="Prepared by synthesis"
/organism="Artificial Sequence"

<220>
<221> MOD_RES
<222> 1..1
<223> ACETYLATION

<400> 75

```
Ser Ile Leu Pro Tyr Pro Tyr Gly Gly Gly Leu Asp Leu Lys Leu Asp
1               5                   10                  15
Leu Lys Leu Asp Leu Lys
            20
```

## Description

### Field of the invention

The present invention concerns the field of functionalized self-assembling peptides suitable for obtaining hydrogels for use in a wide range of applications in the biomedical field, such as for the development of biomaterials for regenerative medicine.

### State of the art

Cell replacement is a crucial step for the regeneration of central nervous system (CNS) lesions, in which endogenous regenerative mechanisms cannot supplant the function of lost cells. One of the most important objectives of regenerative medicine is to obtain standardized and viable cell populations that maintain their phenotype and their biological activities upon transplantation and in *in vitro* cultures. A variety of cells has been used for tissue engineering. Primary cells can be derived from the patient's tissue, avoiding immunological rejection, but in general they are post-mitotic thus featuring a limited proliferative potential; moreover, during the expansion period in culture, the cells can de-differentiate and acquire inappropriate phenotypic characteristics (1). Due to these limitations, stem cells have been studied for their potential in solving most of the drawbacks typical of mature primary cells.

The discovery of the Neural Stem Cells (NSC) (2) with neurogenic capabilities in the developing and adult brain has raised new possibilities for repairing the damaged nervous system (3-5). Regardless of their potential, the success of cell transplantation therapies is not only influenced by the protocols for the isolation and differentiation of stem cells, but also relies on the method by which cells are engrafted and induced to functionally integrate into the affected tissue.

Cells are naturally located in 3-dimensional (3D) microenvironments *in vivo,* where they are surrounded by other cells and by the extracellular matrix (ECM), whose components: collagen, elastin, or laminin, are organized in nanostructures (i.e. fibers, triple helixes, etc.) with specific bioactive motifs that regulate the cell homeostasis.

The potential of success of stem cell transplantation can only be guaranteed by the microenvironment that is created and the presence of a 3D scaffold with optimal motifs, could stimulate differentiation and survival of the specific type of stem cells to be seeded in the scaffold and allow tissue regeneration.

The need and importance is increasingly felt for the development of scaffolds that create synthetic microenvironments providing 3D supports, capable of controlling and directing specific cell-substrate interactions and to warrant proper integration, upon transplantation, of the stem cells within the host tissues.

The object of the present invention is therefore the development of improved self assembling peptides and their use as biomaterials and as scaffolds for tissue regeneration, which overcome the disadvantages related to cell replacement therapies in transplantation.

### Summary of the invention

The present invention concerns a self-assembling peptide (SAP) according to claim 1.

The present invention therefore concerns a novel group of self-assembling peptides (SAPs or SAPeptides), wherein said SAPs are peptides comprising a heptapeptide domain, a spacer region and a backbone region, and are optionally acetylated (Ac) and non-acetylated.

For the purposes of the present invention, each SAP has a corresponding SEQ ID NO., as indicated in the following detailed description.

Advantageously, in a more preferred embodiment, the present invention relates to a SAP selected from the group consisting of:

SEQ ID NO. 16 : $NH_2$-FAQRVPPGGGLDLKLDLKLDLK-$CONH_2$;
SEQ ID NO. 17 : Ac-FAQRVPPGGGLDLKLDLKLDLK-$CONH_2$;
SEQ ID NO. 46 : $NH_2$-FAQRVPPGGGLDLKLDLKLDLK-COOH;
SEQ ID NO. 47 : Ac-FAQRVPPGGGLDLKLDLKLDLK-COOH.

A further aspect of the present invention is a hydrogel comprising the self-assembling peptides according to the present invention and a hydrogelating ingredient.

A still further aspect of the present invention is a self-assembling peptide polymer comprising at least two self-assembling peptides according to the present invention.

In a further embodiment, the invention provides a tabular nanofiber comprising at least two self-assembling peptides according to the present invention.

In a still further embodiment, the invention provides a complex interwoven membrane comprising at least two tabular nanofibres according to the present invention.

A further aspect of the present invention is a self-assembling peptide polymer comprising at least two self-assembling peptides according to the present invention, for use as a medicament.

A further aspect of the present invention is a self-assembling peptide polymer comprising at least two self-assembling peptides according to the present invention, for use as a scaffold in tissue regeneration.

A still further aspect of the present invention is a self-assembling peptide according to the present invention, for use as a biomaterial.

A still further aspect of the present invention is a heptapeptide according to the present invention, for use as a marker for neural stem cells (NSC).

As will be further described in the detailed description of the invention, the self-assembling peptides of the present invention have the advantage of being useful in the development of biomaterials.

**Brief description of the drawings**

The characteristics and advantages of the present invention will be apparent from the detailed description reported below, from the Examples given for illustrative and non-limiting purposes, and from the annexed Figures 1-9, wherein:

Figure 1 (A) and Figure 1 (B) show the effect of the exposure to the chosen heptapeptides over NSC differentiation. In Figure 1 (A) the increment in the neuronal population is quantified. Figure 1(B) depicts differentiated neurons marker for βIII Tubulin obtained from NSC cultured over Cultrex and Laminin substrates when exposed to the heptapeptides described in Example 3.

Figure 2: shows atomic force microscopy (AFM) imaging of a selection of the tested SAPs as described in Example 5. All the synthesized peptides feature a tabular nanofiber morphology consistent with the hypothesis of an effective functionalization at the N-termini preserving the standard cross-p-sheet structure of LDLK12 peptide alone.

Figure 3A: shows frequency sweep tests assessing the storage (G') and loss (G") moduli of a selection of the self-assembled hydrogels according to the present invention as described in Example 6.

Figure 3B: shows frequency sweep tests assessing the storage (G') and loss ( G") moduli of a chosen SAP at different concentrations (1% w/v, 2% w/v, 3% w/v) as described in Example 6.

Figure 4: Secondary structure characterization via Circular Dichroism of a selection of the SAPs as described in Example 7. Spectra have been averaged among three scans per each sample.

Figure 5 (A) and Figure 5 (B) respectively show the morphology of murine and of human NSCs; cultured over a selection of the synthesized SAPs 7 days in vitro (DIV), as described in Example 8.

Figure 5 (C) and Figure 5 (D) are graphs of the proliferation assays for murine and for human NSCs respectively as described in Example 8.

Figure 6: Neural differentiation of murine (A,D) and human neural stem cells (E,H). The quantification of neurons (murine in A; human in E), astrocytes (murine in B; human in F) and oligodendrocytes (murine in C; human in G) is shown; the statistical significances are shown in a table next to each graph, as described in Example 8.

Figure 7: Neural differentiation of murine NSCs over Ac-FAQ self-assembled scaffolds featuring the following stiffnesses: 100 Pa, 500 Pa and 1000 Pa. Neuronal and astroglial populations have been quantified and depicted in Figure 7 (A) and in Figure 7(B) respectively as described in Example 8.

Figure 8(A) Delivery of the biomaterial: spinal cord was exposed at T9-T10 level, then immediately after injury (A1) animals were injected with Ac-FAQ or the vehicle alone using an Hamilton syringe (A2) as described in Example 7.

Figure 8(B) hindlimb locomotor recovery was evaluated in Ac-FAQ treated rats and controls using the 21-points BBB scale as described in Example 9. After 6th week animals treated with Ac-FAQ showed displayed a significantly improved locomotor recovery in than in controls.

Figure 8(C) Measurement of lesion size in saline-injected (C1) and Ac-FAQ-treated (C2) rats was performed on longitudinal sections stained with hematoxylin/eosin as described in Example 10. Scale bar= 800 μm.

Figure 8(D) Measurement of GAP43 positive fibers in saline-injected (D1) and Ac-FAQ-treated (D2) rats was performed on longitudinal sections after immunofluorescence staining as described in Example 10. Scale bar: (D1) and (D2) = 400 μm, (D3) = 200 μm. Nuclei were counterstained with DAPI.

Figure 8(E) Macrophage infiltration in saline-injected (1) and Ac-FAQ-treated (2) rats on immunostained longitudinal sections as described in Example 8. Scale bar: (1) and (2)= 400 μm, (3)= 100 μm. Nuclei were counterstained with DAPI. Values represent means ± SEM. Significance symbols: * p<0.05, ** p<0.005. Arrows indicate the putative presence of the injected SAPs into the cyst.

Figure 9(A) Evaluation of the presence of GAP-43 and βIII positive fibers into the lesion site of Ac-FAQ-treated animals as described in Example 10. Immunofluorescence for GAP-43 (gray arrows) and βIII (white arrows) showed that all GAP-43 positive fibers also expressed βIII (merge image). Most of the GAP-43+ fibers were detected into the cyst (*= cyst) or close to the cyst borders (marked with '+'). Nuclei were counterstained with DAPI.
Figure 9(B) Fibers infiltrating the Ac-FAQ found into the site of injection were positive for both GAP-43 (gray arrows) and βIIITubulin (white arrows). Nuclei were counterstained with DAPI. Scale bars: = 100 μm.

## Detailed description of the invention

The self-assembling peptide (SAP) according to the present invention comprises:

- at the N-terminal region a heptapeptide domain consisting of the sequence:
  -FAQRVPP- SEQ ID N.1
- a spacer region consisting of three consecutive glycine amino acid units; and
- a backbone region consisting of three consecutive LDLK amino acid repetition units at the C-terminal region.

For the purposes of the present invention, the heptapeptide domains or heptapeptides have an aminoacid sequence and a corresponding SEQ ID NO. as follows:

SEQ ID NO. 1 corresponds to the aminoacidic sequence of: FAQRVPP ;
SEQ ID NO. 2 corresponds to the aminoacidic sequence of: QHLPRDH;
SEQ ID NO. 3 corresponds to the aminoacidic sequence of: SSLSVND;
SEQ ID NO. 4 corresponds to the aminoacidic sequence of: YIIPMHD;
SEQ ID NO. 5 corresponds to the aminoacidic sequence of: SLPKLPP;
SEQ ID NO. 6 corresponds to the aminoacidic sequence of: TPLSSHS;
SEQ ID NO. 7 corresponds to the aminoacidic sequence of: SASHWQI;
SEQ ID NO. 8 corresponds to the aminoacidic sequence of: LQAIPRN;
SEQ ID NO. 9 corresponds to the aminoacidic sequence of: YRMPIWP;
SEQ ID NO. 10 corresponds to the aminoacidic sequence of: KLPGWSG;
SEQ ID NO. 11 corresponds to the aminoacidic sequence of: HAIYPRH;
SEQ ID NO. 12 corresponds to the aminoacidic sequence of: GETRAPL;
SEQ ID NO. 13 corresponds to the aminoacidic sequence of: ALTPWAF;
SEQ ID NO. 14 corresponds to the aminoacidic sequence of: GKPMPPM;
SEQ ID NO. 15 corresponds to the aminoacidic sequence of: SILPYPY.

The amino acid sequences defined above use the one letter IUPAC amino acid codes (ie. G corresponds to the amino acid Glycine, P corresponds to the amino acid Proline).

The spacer region comprises three glycine amino acid units (Gly Gly Gly or GGG).

The backbone region comprises three LDLK self-assembling amino acid repetitions or amino acid repetition units or self-assembling amino acid repetition units.

Without being bound to any theory, the inventors believe that SAPs according to the present invention are self-assembling, and therefore undergo spontaneous assembling, due to the self-assembling features of the backbone region.

The SAPs according to the present invention have been identified and linked by the single common inventive concept of being peptides having a predominance of hydrophobic non-polar aminaoacids. SAPs according to the present invention in fact have such a predominance in the heptapeptide domain, in the spacer region which contributes to the conformational flexibility of the SAP and in the backbone region, which due to the strong hydrophobic balance, contributes to the stability and allows to form a nanostructure which can have a larger number of possible functionalizations. The technical relationship which links the SAPs according to the present invention and makes a contribution over the prior art can be seen in the methodology by which the functional motifs have been discovered and in the particular structure of the presently claimed SAPs.

The present invention relates to a SAP wherein the backbone region comprises three LDLK amino acid repetition units.

In the present invention with the term "LDLK amino acid repetition unit" or "LDLK amino acid repetition" is intended the unit which is repeated in the backbone region of the SAP. One LDLK repetition or repetition unit is Leu Asp Leu Lys or LDLK, two LDLK repetition units are the following amino acids: LDLKLDLK.

The backbone region comprises three LDLK aminoacid repetitions (LDLKLDLKLDLK or $(LDLK)_3$).

The LDLK backbone region, featuring a stronger hydrophobic balance, allows for synthesis of a shorter backbone sequence (lowering down production costs and increasing synthesis yield) and for a more stable self-assembled nanostructure, allowing for a larger number of possible functionalizations. In particular the LDLK backbone region consists of the alternation of: one non-polar amino acid, one acid amino acid, one non-polar amino acid and ends with a basic amino acid.

This particular structure of the backbone region has the advantage of allowing the SAPs to easily form hydrogels.

The SAP according to the present invention is a SAP selected from the group consisting of:
X-FAQRVPPGGGLDLKLDLKLDLK-Y; wherein X is $NH_2$ or acetylated (Ac), and wherein Y is $CONH_2$ or COOH.

Advantageously, preferred SAPs of the present invention, have an amino acid sequence and a corresponding SEQ ID NO. chosen from the group comprising:

SEQ ID NO. 16 corresponds to: NH2-FAQRVPPGGGLDLKLDLKLDLK-CONH2;
SEQ ID NO. 17 corresponds to: Ac-FAQRVPPGGGLDLKLDLKLDLK-CONH2;
SEQ ID NO. 46 corresponds to: $NH_2$-FAQRVPPGGGLDLKLDLKLDLK-COOH;
SEQ ID NO. 47 corresponds to: Ac-FAQRVPPGGGLDLKLDLKLDLK-COOH.

As indicated above the amino acid sequences use the one letter IUPAC amino acid codes.

These SAPs are of synthetic origin and are therefore easy to manufacture in large quantities, and they can be modified chemically and biologically. Such modifications allow to construct an ultra-structure promoting cell adhesion and tissue regeneration. The SAPs of the present invention provide the advantages of high biocompatibility which allows them to overcome the problems of tissue rejection, absence of pathogen transfer which is a high risk during implantation, selective and slow drug release due to their 3 dimensional structure and, easy functionalization with bioactive motifs, which therefore allows these SAPs to be very versatile in their use in different applications.

In a further embodiment, these SAPs are peptide-based scaffolds which self-assemble in mild solvent conditions, and easily form hydrogels. Such a hydrogelation is driven by the formation of non-covalent interactions in water, including hydrogen bonding, hydrophobic, electrostatic, and $\pi$-$\pi$ interactions which lead to the formation of organized supramolecular assemblies that can give rise to nanofibers, nanotubes and nanoparticles. Such nanostructured materials also display hydrophilic-hydrophobic residues which provide cross-p-sheet self-assembled nanostructures of easy functionalization and are capable of creating microenvironments suited for triggering tissue regeneration.

These SAPs have the further advantage of being of synthetic origin, and therefore do not have the drawbacks seen in natural peptides such as the tendency to induce inflammatory response and pathogen transfer due to undefined factors that cannot be eliminated by purification prior to implantation, the significant degree of variability between different lots and the difficulty of availability of large scale sources.

Without wishing to be bound to any theory, these SAPs have the advantage of being low molecular weight self-assembling peptides and therefore easier and faster to assemble and to synthesize.

The self-assembling peptide according to the present invention comprises the heptapeptide domain SEQ ID NO. 1.

In a further aspect the present invention provides a self-assembling peptide wherein the N-terminus is optionally modified.

In a still further aspect the present invention provides a self-assembling peptide wherein said N-terminus modification is an acetylation (Ac).

In a further aspect the present invention provides a self-assembling peptide wherein the C-terminus is optionally modified.

In a still further aspect the present invention provides a self-assembling peptide wherein said C-terminus has a $CONH_2$ group.

The invention provides the self-assembling peptides selected from the group consisting of:

$NH_2$-FAQRVPPGGGLDLKLDLKLDLK-$CONH_2$ SEQ ID N.16;
Ac-FAQRVPPGGGLDLKLDLKLDLK-CONH2 SEQ ID N.17;
$NH_2$-FAQRVPPGGGLDLKLDLKLDLK-COOH SEQ ID NO. 46; and
Ac-FAQRVPPGGGLDLKLDLKLDLK-COOH SEQ ID NO. 47.

Preferred SAPs showed an enhanced stimulation of NSC proliferation and enhanced neuronal and oligodendroglial differentiation of mouse neural stem cells (mNSC) and human neural stem cells (hNSCs). Preferred SAPs selected from the group consisting of SEQ ID N.16, SEQ ID N.17, SEQ ID N.46 and SEQ ID N.47, and still more preferably SAPs selected from the group consisting of SEQ ID N.16 and SEQ ID N.17, showed an improved locomotor recovery of acutely injured rats, and did not alter the physiological inflammatory response following SCI, and enhanced nervous regeneration within the malacic nervous tissue *in vivo.*

SAPs according to the present invention therefore enhance transplanted cell survival, stimulate host tissue growth and allow to obtain a controlled release of pro-regenerative cytokines. Moreover SAPs can advantageously be used as hemostat solutions among other applications.

A further aspect of the present invention is a hydrogel comprising the self-assembling peptides according to the present invention and a hydrogelating ingredient.

These functionalized hydrogels, have the potential to become very similar to the fibrous component of the extra cellular matrix, making them the ideal candidates for supporting two and three dimensional cell cultures and fostering human and murine NSCs proliferation and differentiation.

A further aspect of the present invention is that these functionalized hydrogels can be used for enhancing human colon stem cell and human colon cancer stem cell proliferation and/or differentiation in vitro, providing the appropriate physical soft microenvironment and chemical functionalization for cell crypt formation.

A still further aspect of the present invention is a self-assembling peptide polymer comprising at least two self-assembling peptides according to the present invention.

In a further embodiment, the invention provides a tabular nanofiber comprising at least two self-assembling peptides according to the present invention.

In a still further embodiment, the invention provides a complex interwoven membrane comprising at least two tabular nanofibres according to the present invention.

A further aspect of the present invention is a self-assembling peptide polymer comprising at least two self-assembling peptides according to the present invention, for use as a medicament.

A further aspect of the present invention is a self-assembling peptide polymer comprising at least two self-assembling peptides according to the present invention, for use as a scaffold in tissue regeneration.

The SAPs according to the present invention could be advantageously used in the development of the complex polymers for effective tissue engineering and for the development of biomaterials and biological prostheses to assist or enhance motor control lost by trauma, disease, or defect.

The self-assembling peptide polymer according to the present invention, for use as a scaffold in tissue regeneration wherein said tissue is a tissue of the CNS.

Tissues of the CNS such as nerves, can be damaged through trauma which may lead to the severance of nerves or disease, such as stroke, multiple sclerosis, diabetes, spina bifida, and polio. The most dramatic and serious nerve damage occurs to the spinal cord.

As there is no cure for nerve damage, the SAPs of the present invention are advantageously used in developing tailored scaffolds for a regenerative medicine applications such as regeneration of spinal cord injury, and for nerve regeneration.

A still further aspect of the present invention is a self-assembling peptide according to the present invention, for use as a biomaterial.

Without being bound to any theory, a biomaterial is any matter, surface, or construct that interacts with biological systems or a substance that has been engineered to take a form which, alone or as part of a complex system, is used to direct, by control of interactions with components of living systems, the course of any therapeutic or diagnostic procedure.

A still further aspect of the present invention is a heptapeptide - FAQRVPP- of SEQ ID N.1, for use as a marker for neural stem cells (NSC).

Markers are frequently used to characterize stem cell populations and provide a useful tool for initially identifying as well as isolating stem cells. The heptapeptides (functional motifs) according to the present invention have been proved to identify neural stem cell populations, useful quality for stem cell research and regenerative medicine.

EXAMPLES

**Example 1.**

Neural stem cell proliferation and differentiation

Murine and Human neural precursor cultures are established and expanded as previously described (6) Egf-Generated Cns Progenitor Cells. Neuron 1993,11, 951-66).

Murine neural precursors isolated from the sub ventricular zone (SVZ) of 8-week-old CD-1 albino mice striata, at passage 10, were used. Human NSCs were isolated from the central nervous system, in particular the diencephalon and the cerebral cortex of human brain 10.5 weeks from conception as previously described 43. The modalities for obtaining the primary tissue are in agreement with the guidelines of the European Network for Transplantation (NECTAR).

Cell proliferation (both for human and murine neural stem cells) was performed in NS-A serum-free medium (Euroclone, Irvine, UK), in the presence of basic fibroblast growth factor ($\beta$FGF from PeproTech, Rocky Hill, NJ) and epidermal growth factor (EGF from PeproTech) at final concentrations of 10 ng/ml and 20 ng/ml. The medium without growth factors was used as a basal medium.

Bulk cultures of mouse and human NSCs were generated by mechanically dissociating neurospheres and plating cells in untreated flasks at the appropriate density ($1 \times 10^4$ cells/cm$^2$) every 4-5 days in the same growth medium. Cell counting and viability was performed at every passage, using trypan blue exclusion.

**Example 2.**

Phage biopanning

Two days prior cell seeding murine NSCs were mechanically dissociated in order to seed the maximum percentage of stem cells.

NSCs, three million per plate, were seeded over Laminin coated 10 cm Petri dishes (Falcon). After an overnight incubation in 10ml of βFGF supplemented (10 ng/ml) neural basal media, cells were washed with pre-warmed Incubation Medium.

The Ph.D.-7 phage library was used (New England Biolabs), composed of phage clones with random 7-mer peptides fused to the N-terminal of PIII, a minor coat protein of M13 phage.

Before biopanning experiments against NSCs in adhesion, the phage library was incubated in a Laminin-coated tissue culture dish in order to remove phages with affinity to the Laminin coated plastic surface (negative selection). Mouse Laminin (10 mL at 25 μg/ml concentration) (Roche) was incubated overnight at 37°C. $10^{12}$ cfu of phages were incubated for one hour at 37°C in pre-warmed Incubation Medium (DMEM supplemented with 0.1% BSA) in Laminin-coated plastic surfaces (setting for adhering NSCs) or plain plastic surfaces (setting for NSCs in suspension). Then incubation Medium and a total volume of five washes of Washing Buffer (DPBS supplemented with 1% BSA and 0.05% Tween-20) were collected and eluted phages were amplified as described in the standard protocol of New England Biolabs.

$10^{12}$ cfu of the negatively selected phage library were diluted in 10 ml Incubation Medium and pipetted onto the adhering NSCs or NSCs in suspension.

After 1 hour of incubation at 37°C cells were washed five times with pre-warmed Washing Buffer, cell membrane bound phages (MP) and internalized clones (IP) were recovered from independent duplicates of differentiating progenies by eluting and/or lysing the cells respectively. The cell surface was stripped by a 5 minutes incubation of 5 ml of Glycine Buffer (0.2 M Glycine-HCl supplemented with 1 mg/ml BSA (pH 2.2)) at RT. Eluted Phages were neutralized with 5 ml of 1 M Tris-HCl buffer (pH 8) and amplified.

Cells were then lysed with 9 ml of Lysing Buffer (0.1% TEA in PBS) for 5 minutes at RT. Internalized phages were neutralized with 1 ml of 1 M Tris-HCl buffer.

Neutralized phages were titered by infection of Escherichia coli to monitor the selection. Internalized and eluted phages were amplified separately for another round of selection. Three rounds of positive selection were performed. At round number two and three the biopannings of internalized and eluted phages were performed in different cell cultures (but with identical culture conditions) by collecting the phages of interest (i.e. internalized phages from libraries of internalized phages) and discarding the leftovers.

After the third round of biopanning, phage clones were randomly picked out from the titer plates and sequenced. Thirty phage plaques per each set of phages were randomly picked for sequencing. A small number of clones wasn't successfully sequenced. The highest consensus was shown for FAQRVPP (53.5% for IP and 62.5% for MP), QHLPRDH (14.2% for IP and 8.3% for MP) and SSLSVND (10.7% for IP and 4.2% for MP) detected in both IP and MP phages as can be seen in Table 1A. In the case of panning against NSC in suspension the highest consensus was for KLPGWSG (47.36 for MP), GETRAPL (14.28% for IP and 10.52% for MP) and HAIYPRH (28.57% for IP and 5.26% for MP).

TABLE 1A:

| Internalized phage clones | | | Eluted phage clones | | |
|---|---|---|---|---|---|
| Sequence | Frequency | % | Sequence | Frequency | % |
| FAQRVPP | 15 | 53.5 | FAQRVPP | 15 | 62.5 |
| QHLPRDH | 4 | 14.2 | QHLPRDH | 2 | 8.3 |
| SSLSVND | 3 | 10.7 | SSLSVND | 1 | 4.2 |
| YIIPMHD | 1 | 3.5 | YIIPMHD | 1 | 4.2 |
| SLPKLPP | 1 | 3.5 | SSELVTH | 1 | 4.2 |
| TPLSSHS | 1 | 3.5 | TAVNSDA | 1 | 4.2 |
| SASHWQI | 1 | 3.5 | TPPFAAW | 1 | 4.2 |
| LQAIPRN | 1 | 3.5 | TTTPTTP | 1 | 4.2 |
| YRMPIWP | 1 | 3.5 | LTTGSGS | 1 | 4.2 |

Table 1B shows the sequences of the phages recovered after three rounds of biopanning of NSCs cultured in suspension.

| Internalized phage clones | | | Eluted phage clones | | |
|---|---|---|---|---|---|
| **Sequence** | **Frequency** | **%** | **Sequence** | **Frequency** | **%** |
| HAIYPRH | 4 | 28.57 | KLPGWSG | 9 | 47.36 |
| GETRAPL | 2 | 14.28 | HAIYPRH | 1 | 5.26 |
| SILPYPY | 2 | 14.28 | GETRAPL | 2 | 10.52 |
| | | | ALTPWAF | 2 | 10.52 |
| | | | GKPMPPM | 2 | 10.52 |

For homology identification the primary peptide sequences were compared to the NCBI database using the BLAST program to align short, nearly exact matches.

No full homological protein sequences were identified, however some potentially relevant homologies are shown in Table 2A and Table 2B: ranging from a portion of the Microtubule-affinity regulating kinase 1 (MARK1), relevant in neuronal differentiation, in the case of FAQ, to the mitochondria precursor of intermembrane enzyme Sulfite Oxidase, whose deficiency gives neurological abnormalities fatal at early age, in the case of QHL, to the Activine A Type 1 Receptor, influencing the neuronal differentiation in some cell lines, for SSL. On the other hand, fractions of KLP were found in several FGF-like domains of MEGF10 protein, predominantly expressed in the brain and known to function as a phagocytic receptor, while GET showed a relevant homology for the Cadherin EGF G-type receptor 2.

Table 2A:

| Peptide Sequence | Protein | Accession |
|---|---|---|
| FAQRVPP | Microtubule Affinity-Regulating Kinase MARK1 (581-585) | NP_663490 |
| QHLPRDH | Sulphite Oxidase, Mitochondrial Precursor (356-362) | NP_776094 |
| SSLSVND | Activine A, Receptor Type 1 (55-61) | CAM15401 |

*Potentially relevant sequence homologies identified by BLAST in mouse: underlined amino acids stand for exact matches with known peptide sequences.*

Table 2B:

| Peptide Sequence | Protein | Accession |
|---|---|---|
| KLPGWSG | MEGF10 (561-565), Notch1 | Q6DIB5.1 |
| GETRAPL | Cadherin EGF Receptor 2 (5-10), Chordin | XP_537042.2 |
| HAIYPRH | Neurocon Precursor, Glucosylceramidase (210-215) | Q69ZF3.2 |

**Example 3.**

Neurogenic effect of #KLP, #GET and #HAI sequences over NSC cultures in vitro.

The following sequences were synthesized as listed in Table 3 to assess their effect over NSC differentiation.

Table 3:

| Peptides Sequence | Acronym |
|---|---|
| $NH_2$-KLPGWSG-$CONH_2$ | #KLP |
| $NH_4$-GETRAPL-$CONH_2$ | #GET |
| $NH_2$-HAIYPRH-$CONH_2$ | #HAI |

Peptides of the present section were synthesized on a 0.1 mmol scale with standard fluorenylmethoxycarbonyl solid-phase techniques using a CEM Liberty automated microwave peptide synthesizer. MBHA rink amide resin (0.5 mmol/g substitution) was used to produce C-terminal amides; activation was performed with 0.5 M HOBt/HBTU in DMF. Peptides were then cleaved from the resin and deprotected with 9ml of 95% trifluoroacetic acid (TFA), 2,5% water and 2,5% triisopropylsilane. The cleaved peptides were precipitated, washed several times with cold diethyl ether and dissolved in 20-25% of acetonitrile solution prior to be lyophilized and stored at -20 °C. Crude peptides were analyzed and purified by reverse phase HPLC using a Waters system equipped with an analitycal and semi-preparative BioBasic C4 (Thermo Scientific, UK). Eluents were 0,1% (v/v) TFA in water (Buffer A) and 0,1% (v/v) TFA in acetonitrile (Buffer B). Identity of the peptides was confirmed with MALDI-TOF mass spectrometry. After purification, 0,1 M HCl exchange was performed at a volume of 1:1 in order to extract residual TFA. Then, peptides were lyophilized again.

*In vitro* tests were performed as previously described (8), in order to assess whether the heptapeptides obtained of the present invention influence NSCs differentiation.

Murine cells (at a concentration of $2 \times 10^4$ cells/cm$^2$) were seeded in each well (96 multi-well plate) previously coated with Cultrex-BME® substrate (R&D systems) (1:100 dilution in basal medium, overnight incubation) or with Laminin (Roche) (1:5 dilution in PBS 1x, pH 7.4, overnight incubation).

Initially, cells were cultured with basal medium supplemented with βFGF (10 ng/ml), added to enhance neuronal progeny differentiation. At 3 days *in vitro* (DIV), βFGF medium was replaced with Leukemia Inhibitory Factor (LIF, Chemicon) (20 ng/ml) and Brain Derived Neurotrophic Factor (BDNF, Peprotech) (20 ng/ml). Fresh medium was added every three days. The chosen peptides were dissolved in all cell culture media (but not in the coatings) at a concentration of 0.1 mg/ml.

Immunofluorescence tests were performed for assaying the phenotypes of the differentiated murine NSC progenies. The following antibodies were used: rabbit polyclonal against GFAP (1:500, DakoCytomation) for astrocytes and mouse monoclonal against βIII-Tubulin (1:500, Covance) for neurons. Secondary Abs were Goat anti Mouse Alexa 488 (1:1000, Molecular Probes), Goat anti Mouse Cy3 (1:1000, Jackson Immunoresearch), Cell nuclei were counterstained with DAPI (Molecular Probes). Quantitative analyses were performed by counting 100-300 cells for each of 9 randomly chosen fields over a total of 3 independent experiments.

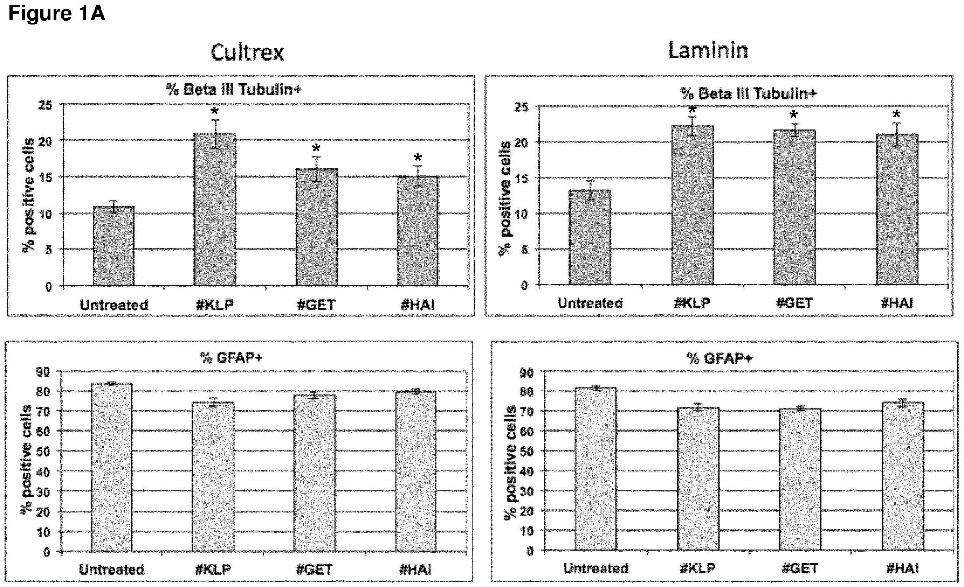

Values, reported as means ± standard error of the mean (Figure 1A).

Results:

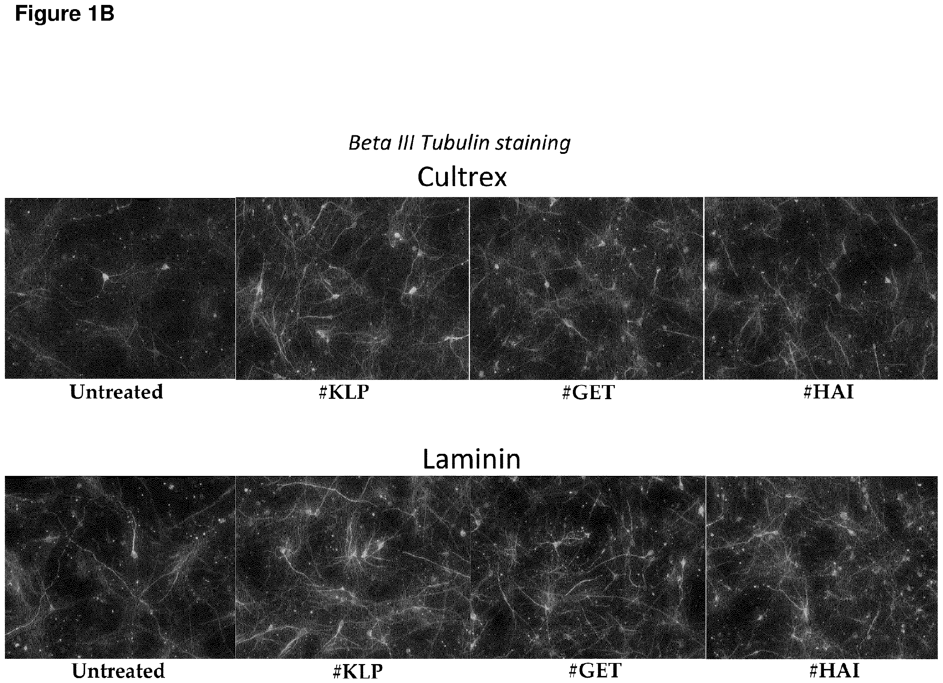

At 12 days in vitro (DIV) differentiated mouse NSC showed a significant increase in the percentage of differentiated neurons (Beta III Tubulin staining) in both Cultrex and Laminin coated wells upon exposure to #KLP, #GET and #HAI, at the expenses of the percentage of differentiated astorcytes (GFAP staining). Neuronal increase was significant (* p<0.05) in all treated NSCs vs untreated NSCs. Highest increase of the neuronal phenotype was detected for #KLP in both cases. These results show that the discovered peptides can influence NSC differentiation toward the neural phenotype (Figure 1B).

**Example 4.**

Self-assembling peptide functionalization and purification

The SAPs according to the present invention were synthesized as listed in

Table 4:

| Functionalized SAPeptides | Acronym |
|---|---|
| NH$_2$-FAQRVPP-GGG-LDLKLDLKLDLK-CONH$_2$ | FAQ |
| Ac-FAQRVPP-GGG-LDLKLDLKLDLK-CONH$_2$ | Ac-FAQ |
| NH$_2$-QHLPRDH-GGG-LDLKLDLKLDLK-CONH$_2$ | QHL |
| Ac-QHLPRDH-GGG-LDLKLDLKLDLK-CONH$_2$ | Ac-QHL |
| NH$_2$-SSLSVND-GGG-LDLKLDLKLDLK-CONH$_2$ | SSL |
| Ac-SSLSVND-GGG-LDLKLDLKLDLK-CONH$_2$ | Ac-SSL |
| NH$_2$-KLPGWSG-GGG-LDLKLDLKLDLK-CONH$_2$ | KLP |
| Ac-KLPGWSG-GGG-LDLKLDLKLDLK-CONH$_2$ | Ac-KLP |

The must recurrent clone sequences were synthesized as functional motifs linked, via a 3-glycines spacer, to the N-termini of a self-assembling peptide LDLKLDLKLDLK backbone sequence, proven to spontaneously fold in β-sheets upon exposure to neutral pH solutions giving injectable hydrogels as follows:

Ac-1234567-GGG-LDLKLDLKLDLK-CONH2

NH$_2$-1234567-GGG-LDLKLDLKLDLK-CONH$_2$

Wherein starting from the N-termini: Ac is an acetyl group for improving peptide stability and NH$_2$ is an ammine group of the original sequences, 1234567 is a hepta-peptide, GGG is a spacer comprising three Glycine peptides, LDLKLDLKLDLK or (LDLK)$_3$, CONH$_2$ is a carbonyl functional group located at the C-termini.

Without being bound to any theory, self-assembling peptides of the present invention may also be synthesized in the reverse order:

Ac- LDLKLDLKLDLK -GGG- 1234567-CONH2

NH$_2$-LDLKLDLKLDLK-GGG-1234567-CONH$_2$

Self-assembling peptides of the present invention were synthesized on a 0.1 mmol scale with standard fluorenylmethoxycarbonyl solid-phase techniques using a CEM Liberty automated microwave peptide synthesizer. MBHA rink amide resin (0.5 mmol/g substitution) was used to produce C-terminal amides; activation was performed with 0.5 M HOBt/HBTU in DMF. 47 and 49 were acetylated using 20% acethic anhydride solution in DMF. Peptides were then cleaved from the resin and deprotected with 9ml of 95% trifluoroacetic acid (TFA), 2,5% water and 2,5% triisopropylsilane. The cleaved peptides were precipitated, washed several times with cold diethyl ether and dissolved in 20-25% of acetonitrile solution prior to be lyophilized and stored at -20 °C. Crude peptides were analyzed and purified by reverse phase HPLC using a Waters system equipped with an analitycal and semi-preparative BioBasic C4 (Thermo Scientific, UK). Eluents were 0,1% (v/v) TFA in water (Buffer A) and 0,1% (v/v) TFA in acetonitrile (Buffer B). Starting conditions were 10% buffer A and 90% buffer B and the gradient developed with a linear increase in buffer B. In 15 minutes gradient went to 65% buffer B. Identity of the peptides was confirmed with MALDI-TOF mass spectrometry. After purification, 0,1 M HCI exchange was performed at a volume of 1:1 in order to extract residual TFA. Then, peptides were lyophilized again. The peptides were successfully synthesized and purified (purity >95%).

**Example 5.**

Atomic Force Microscope imaging and measurements

Self-assembling peptides according to the invention were dissolved in distilled water (GIBCO), at a concentration of 1% w/v one day prior imaging. The day after peptide solutions were diluted (in a ratio of 1:50), 2ml of these solutions were placed on mica muscovite substrates and kept at room temperature for 2 minutes. The mica surfaces were then rinsed with distilled water to remove loosely bound peptides and solution was let to evaporate for 30 minutes.AFM images were collected in tapping™ mode by a MultiMode Nanoscope IIIa (Digital Instruments) using single-beam silicon cantilever probes (Veeco RTESP: resonance frequency 300 KHz, nominal tip radius of curvature 10nm, forces constant 40 N/m). Measured fiber dimensions were corrected because of the tip convolution. Being the observed tabular nanofiber heights (between 1 and 1.5 nm) far lower than the tip radius (10nm) the observed widths were corrected with the formula 15:

$$\Delta x = \sqrt{2\left[h\left(2r_t - h\right)\right]}$$

Where $\Delta x$ is the width broadening effect, h is the nanofiber height, and rt is for tip radius.

Results are presented as means $\pm$ standard error of the mean.

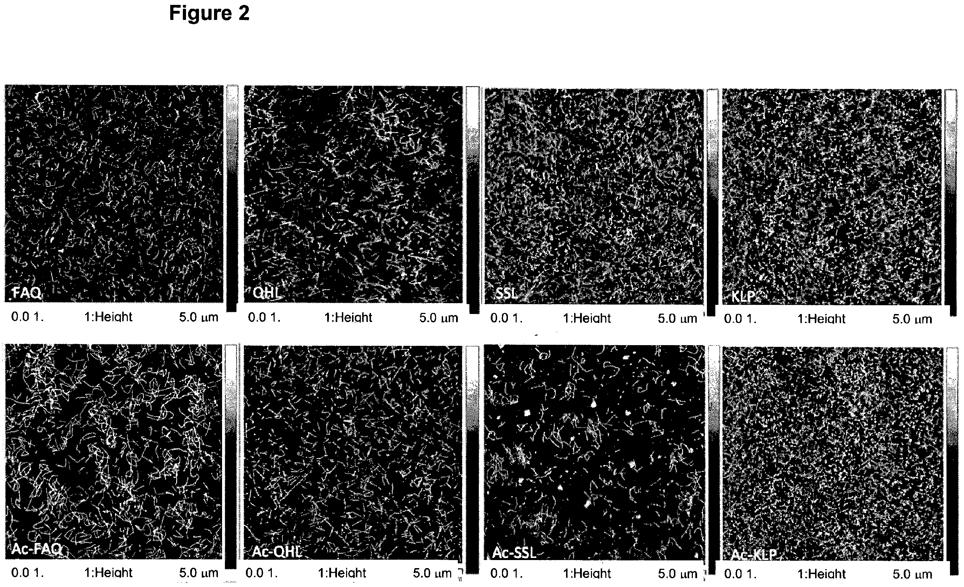

Atomic force microscope (AFM) measurements revealed that all peptides form tabular nanofibers (see Figure 2). Deconvolved measurements are reported in Table 4. Processed data are consistent with the usual double layered antiparallel β-sheet model described for these functionalized SAPeptides, giving, for these sequences, -12 nm wide and ~1.6 nm high nanofibers.

The SAPs of the present invention self-assembled into similar nanofibers, a selection of these SAPs are shown in Table 5 below:

| SAPeptide | Height | Width |
|---|---|---|
| FAQ | 1.66 nm$\pm$ 0.12 nm | 13.4 nm $\pm$1.36 nm |
| Ac-FAQ | 1.52nm$\pm$ 0.13 nm | 13.76 nm$\pm$1.12 nm |
| QHL | 1.77 nm$\pm$ 0.13 nm | 12.27 nm$\pm$1.17 nm |

(continued)

| SAPeptide | Height | Width |
|---|---|---|
| Ac-QHL | 1.7 nm± 0.17 nm | 12.54nm±1.35 nm |
| SSL | 1.57 nm± 0.14 nm | 13.6 nm±1.58 nm |
| Ac-SSL | 1.74 nm± 0.27 nm | 12.3 nm±1.53 nm |
| KLP | 1.70 nm± 0.07 nm | 14.78 nm± 0.67 nm |
| Ac-KLP | 1.76 nm± 0.07 nm | 14.66 nm± 0.55 nm |

**Example 6.**

Rheology

Rheological properties of the self-assembling peptides of the invention were determined using a controlled stress TA Instruments AR-2000ex rheometer (TA Instruments). A cone-and-plate geometry (acrylic cone diameter, 20 mm; angle, 1°; truncation gap 34 $\mu$m) was used. Preliminary strain sweeps were performed for each sample to define the linear viscoelastic region, thus ensuring that moduli were independent of strain. Time sweeps, after addition of PBS (1x) were recorded at 25 C (angular frequency $\omega$=1 Hz) and continued till G' plateaued. Frequency sweeps, both for peptide water solutions and for self-assembled scaffolds, were performed at 25 C with the instrument in oscillatory mode at controlled strain of 1 %. Final strain sweeps was performed at 1Hz oscillatory frequency and at 25 C: onset point of failure was calculated by linear interpolation and subsequent determination of the intersection of the G' modes within the linear region and at material failure respectively.

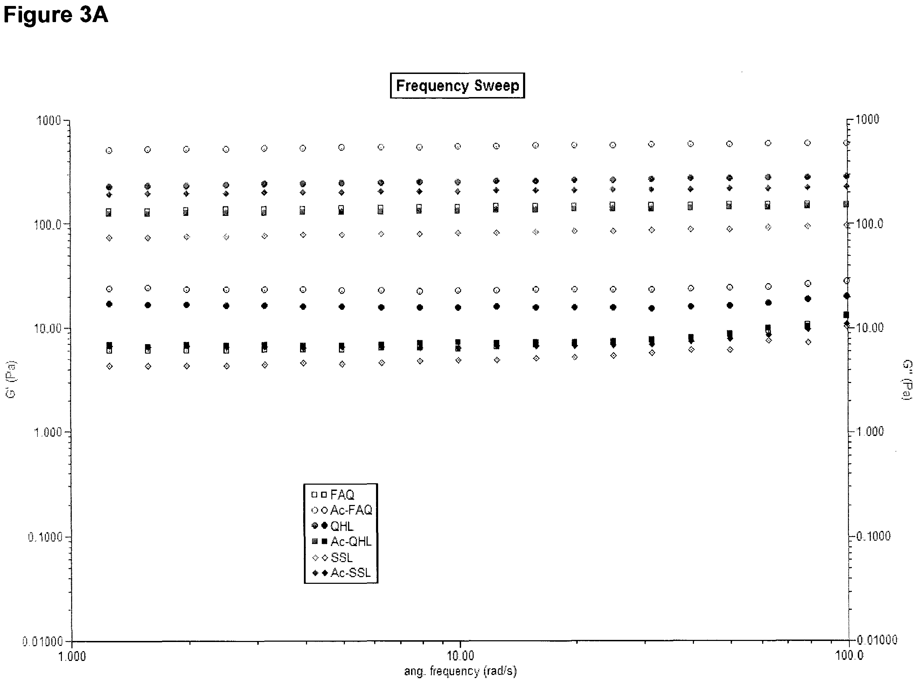

The synthesized peptides self-assembled (at 1% w/v concentration solutions) (1x, pH 7.4) and formed solid scaffolds after addition of PBS. In the rheology tests (Figure 3A) the storage modulus (G') was linear in the 1-100 Hz range (frequency sweep test), and well above the loss modulus (G"): a classic pattern seen in solid structures.

Linear values (in the 1-100 Hz range) of the assembled scaffolds reside in the 70-400 Pa and 8-20 Pa ranges respectively. These responses are typical of solid soft hydrogels.

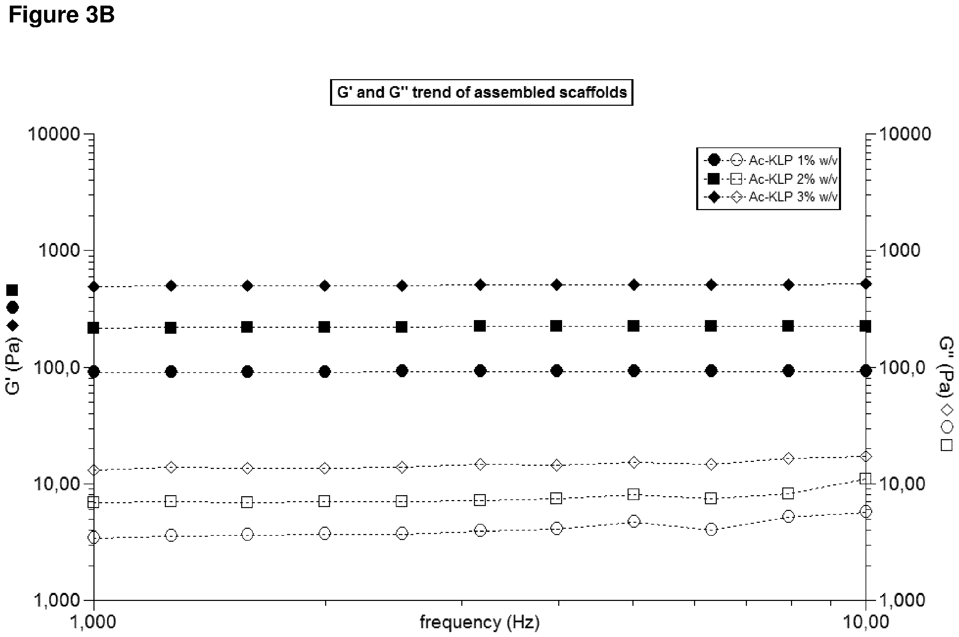

In particular, for peptide Ac-KLP, we assessed G' and G" values at different concentrations (i.e. 1% w/v, 2% w/v and 3% w/v) as depicted in Figure 3B. G values (both G' and G") increased with concentration increments. This could be useful to obtain hydrogels with stiffnesses similar to those of the tissues to be regenerated or to favor neural differentiation of NSCs (favored in softer scaffolds).

Results:

The gelation results of a selection of the SAPs (dissolved at 1% w/v) according to the present invention is listed in Table 6:

| SAPeptide | G' pre-assembling | G' post-assembling | Onset point (% strain) |
|---|---|---|---|
| FAQ | 6.66 Pa | 142.7 Pa | 25.43 % |
| Ac-FAQ | 5.11 Pa | 551.9 Pa | 22.75 % |
| QHL | 4.53 Pa | 296.9 Pa | 28.58 % |
| Ac-QHL | 2.68 Pa | 166.7 Pa | 10.16 % |
| SSL | 9.01 Pa | 124.7 Pa | 17.78 % |
| Ac-SSL | 6.72 Pa | 267.3 Pa | 26.12 % |
| KLP | 3.30 Pa | 51.38 Pa | 56.46 % |
| Ac-KLP | 2.88 Pa | 91.2 Pa | 47.82 % |

*Average values of the storage moduli of SAPeptide solutions (G' pre-assembling), jellified scaffolds (G'post-assembling) and strain percentage at rupture (Onset point).*

In Table 6 average G' values are reported (1-100 Hz range) before and after the addition of PBS. Onset points of rupture (strain sweep tests) are in the range of 10%-30% of strain. Thus the addition of the chosen functional motifs at the N-termini did not impair the capability of forming hydrogels of LDLK-12. Notably, all of the G' values are in the 100-1000 Pa range, reported as typical stiffness of nervous tissue, and favor neural differentiation of mesenchymal stem cells *in vitro.*

**Example 7.**

Molecular structure characterization: circular dichroism (CD) analysis

Far-UV CD spectra of each of the peptides of the present invention are recorded between 190nm - 260nm at room temperature on an Aviv 62DS spectrometer. All measurements were carried out in 1-mm quartz cuvette in distilled water. Spectra are from accumulation of 3 scans. Blank spectra of the buffer without sample are subtracted. SAPeptide solutions were dissolved in water at 1% w/v and diluted at a final concentration of 0.2 mg/ml at one day after dissolution in water. Spectra are recorded in 2-nm steps, averaged over 4 seconds and normalized into Delta Epsilon units. Protein structures were deconvolved by using the CD Spectra Deconvolution Software 2.1 using 33 base spectra of known proteins.

Results:

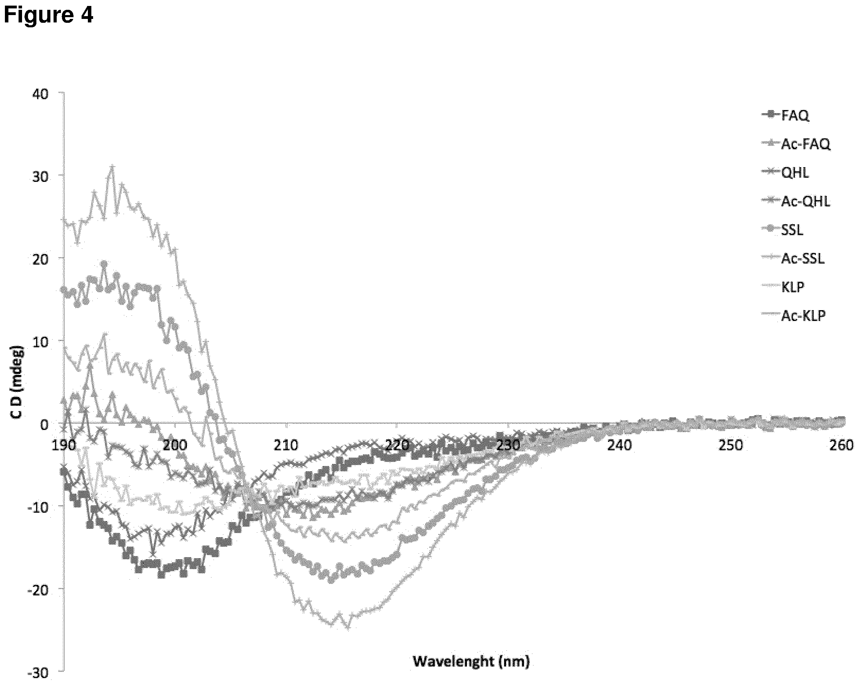

AFM findings, suggesting that all SAPeptides self-assemble mainly into β-sheets, are confirmed by CD spectra (Figure 4): indeed a minimum value of 45% of β-sheets was detected in all SAPeptides after spectra deconvolution. Nonetheless, acetylated functional motifs showed spectra characterized by a higher percentage of β-structures with respect to their unacetylated (and more charged) counterparts (Figure 4), suggesting a tangible effect of the net charge at the free N-terminus over the stability of the assembled molecular structures (7)

**Example 8.**

*In vitro* tests: imaging and cell proliferation assay

*In vitro* tests were performed as previously described (8), in order to assess whether the biomaterials (assembled hydrogels) obtained with the SAPs of the present invention influenced NSCs adhesion, survival and/or differentiation. SAPeptides (30 μl for each well at a concentration of 10 mg/ml) were assembled into 96 multiwells two hours before cell. Cells (at a concentration of $2 \times 10^4$ cells/cm$^2$) were seeded on the top-surface of each assembled scaffold. Initially, cells were cultured with basal medium supplemented with βFGF (10 ng/ml), added to enhance neuronal progeny differentiation. At 3 days *in vitro* (DIV), βFGF medium was replaced with Leukemia Inhibitory Factor (LIF, Chemicon) (20 ng/ml) and Brain Derived Neurotrophic Factor (BDNF, Peprotech) (20 ng/ml). Fresh medium was added every three days. For both viability and differentiation assays positive and negative controls consisted of Cultrex-BME® substrate (R&D systems) (1:100 dilution in basal medium) and untreated bottom well surfaces respectively. Cell viability was quantified via CellTiter 96® Aqueous Proliferation Assay (Promega) at 7 DIV as recommended in the Promega protocol. After calibrating the linear response between the cell number and absorbance values, proliferated cell populations were quantified (n=5) by using a Vmax microplate reader (Molecular Devices) at 490nm wavelength. Values, reported as means ± standard error of the mean, were blanked to their respective controls consisting of same substrates and cell culture media without cells.

Results:

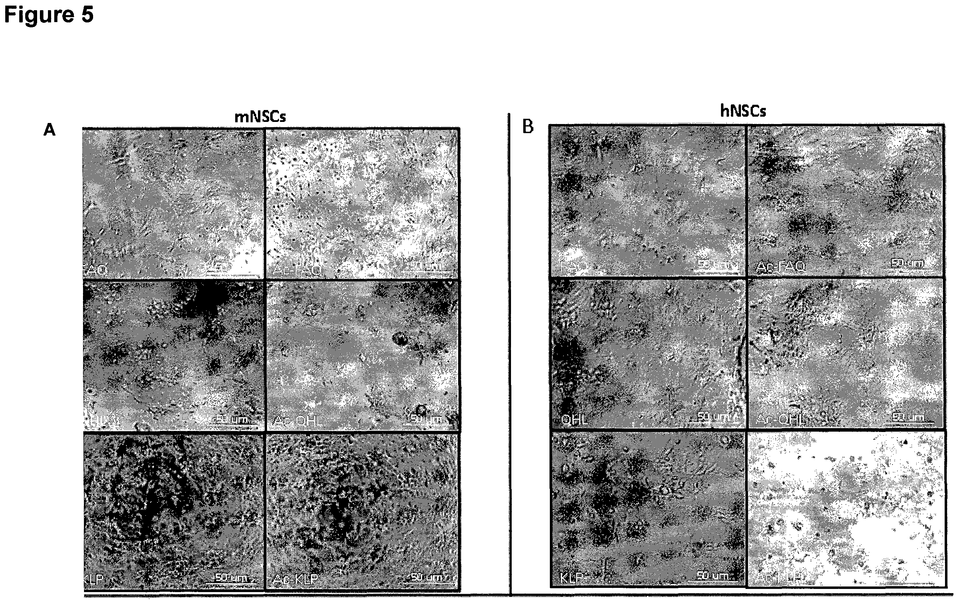

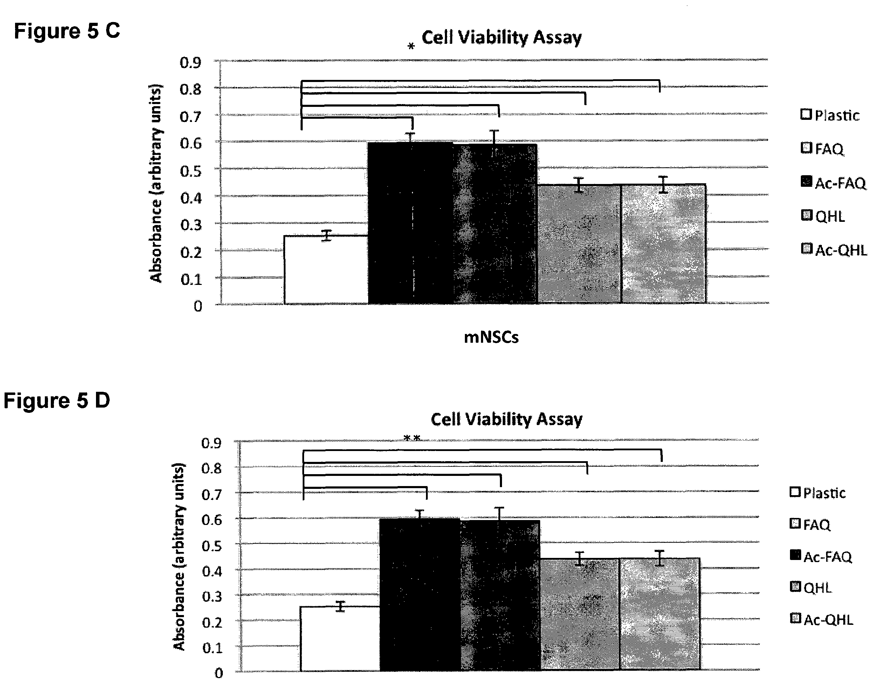

After 7 days in vitro (DIV) both mouse NSCs and human NSCs progenies cultured over assembled hydrogels of FAQ and Ac-FAQ showed spread and branched cells, evenly covering the scaffolds top surfaces (figure 5A and B). No appreciable differences in cell spreading or branching were seen among the acetylated and not-acetylated peptide counterparts. Murine and human NSCs gave similar results. Wide spreading was detected for FAQ. FAQ and QHL, both acetylated and not, significantly fostered cell viability with respect to control (* P<0.005 for murine NSCs, ** P<0.03 for human NSCs; t-student paired test: n=5).
MTS cell viability assays (figure 5C and D) confirmed the qualitative morphological observations: mouse and human NSCs showed comparable results, i.e. cell population was bigger for FAQ and Ac-FAQ, followed by QHL and Ac-QHL. No significant difference was detected between Acetylated and not Acetylated counterparts, while FAQ and QHL significantly favored both murine and human NSCs cell viability in respect to control. FAQ and QHL showed to be functionalized SAPs effective in obtaining NSC survival and proliferation. KLP functionalized SAP (both Acetylated and not Acetylated) formed solid hydrogels and fostered NSCs adhesion and spreading similarly to FAQ.

Immunofluorescence

Immunofluorescence tests were performed for assaying the phenotypes of the differentiated human and murine NSC progenies. In the case of KLP and Ac-KLP just murine NSC have been used. Ac-KLP and KLP scaffolds were prepared at different concentrations in order to display three different stiffness values (viz. 100, 500 and 1000 Pa). This was done to assess the influence of scaffold concentration (and consequently its stiffness) over NSC differentiation. All the other peptides were tested at 1% v/w concentration instead. The following antibodies were used: rabbit polyclonal against GFAP (1:500, DakoCytomation) for astrocytes, mouse monoclonal against βIII-Tubulin (1:500, Covance) for neurons, mouse monoclonal against GalC (1:200, Chemicon) and O4 (1:200, Chemicon) for oligodendrocyes. Secondary Abs were Goat anti Mouse Alexa 488 (1:1000, Molecular Probes), Goat anti Mouse Cy3 (1:1000, Jackson Immunoresearch), Goat anti Rabbit Cy3 (1:1000, Jackson Immunoresearch). Cell nuclei were counterstained with DAPI (Molecular Probes). Quantitative analyses were performed by counting 100-300 cells for each of 9 randomly chosen fields over a total of 3 independent experiments. Phase contrast and fluorescence images of the adhering cells were acquired via Zeiss microscopes Axioplan 2 and ApoTome System at one week after seeding.

Results:

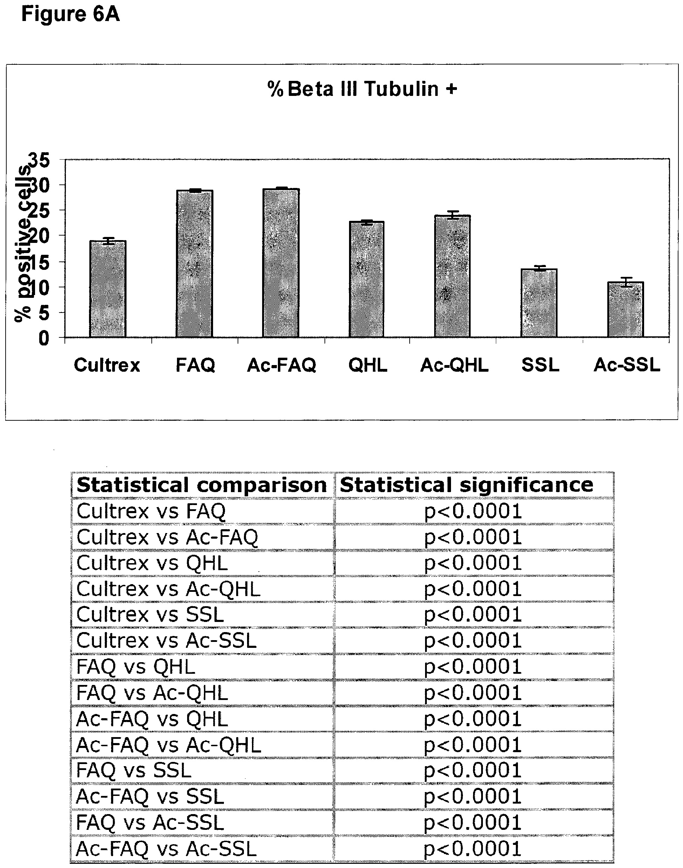

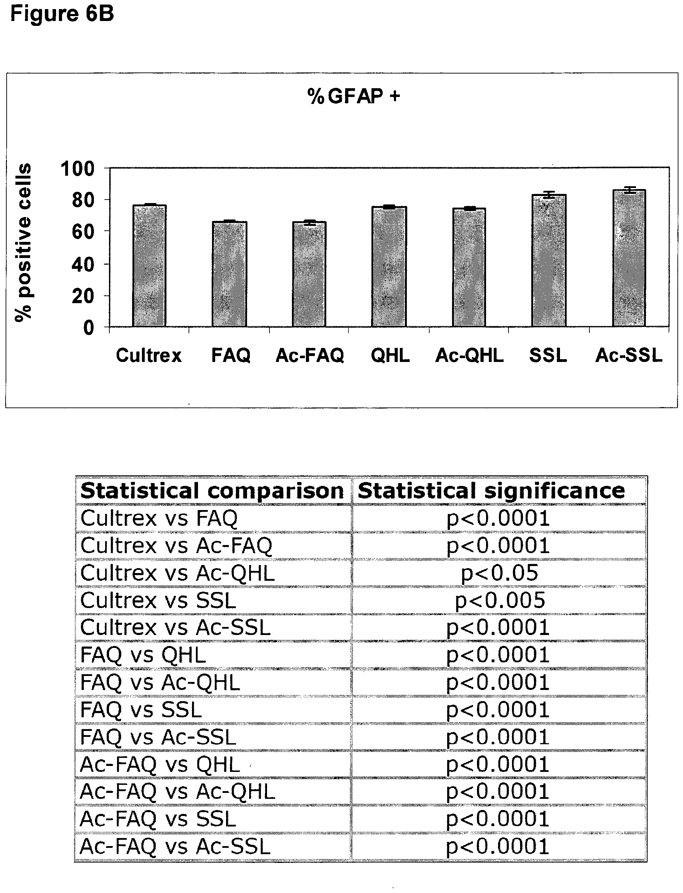

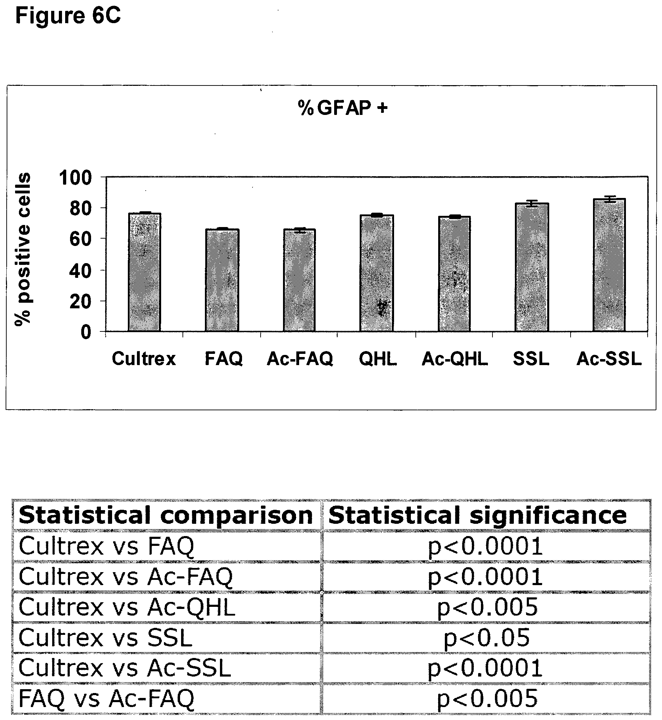

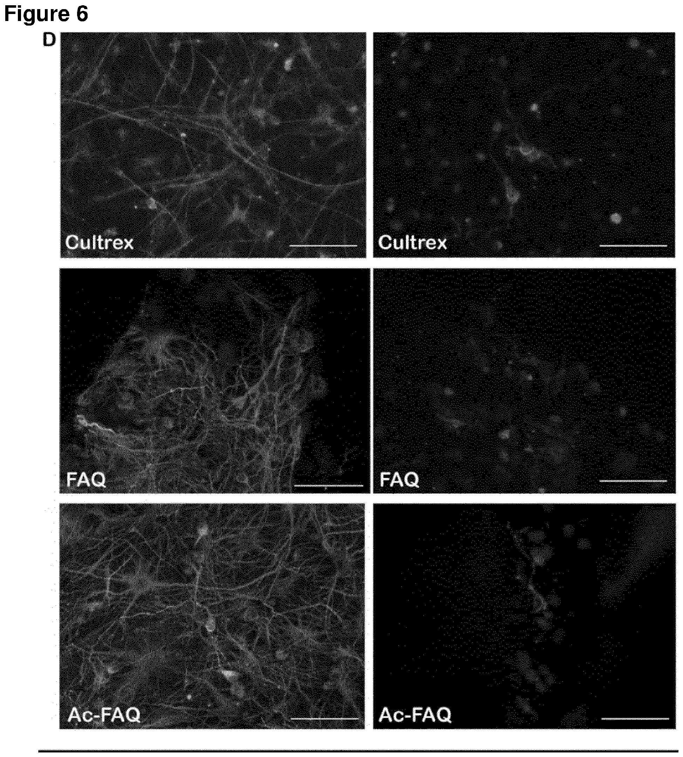

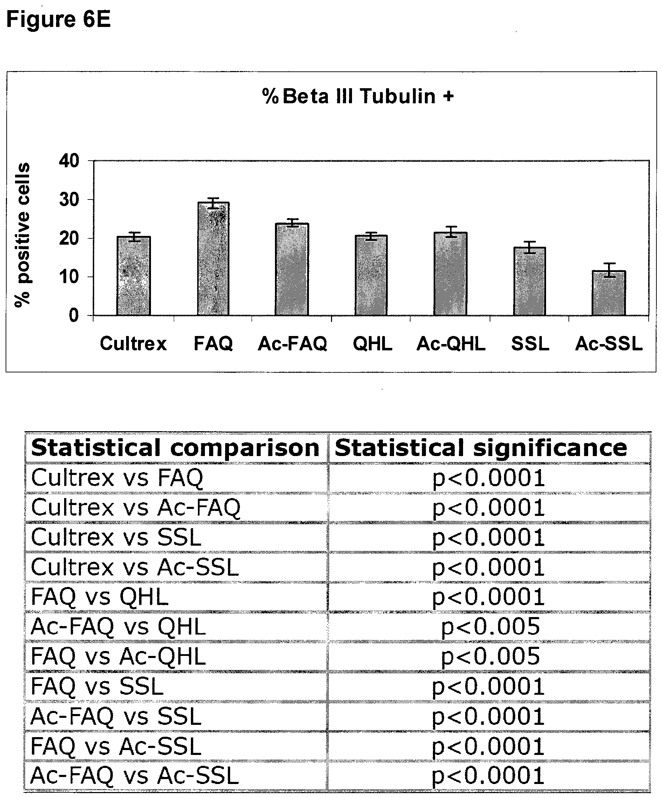

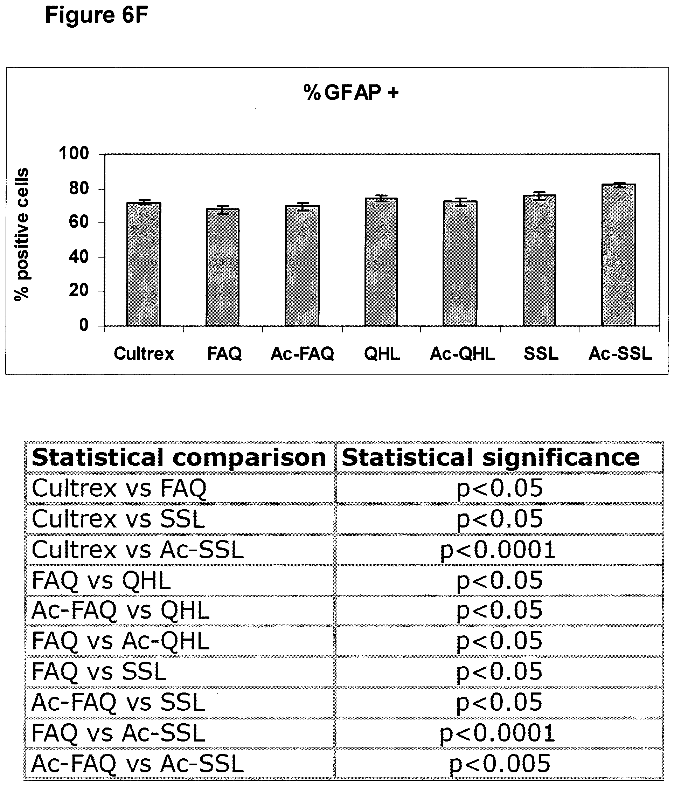

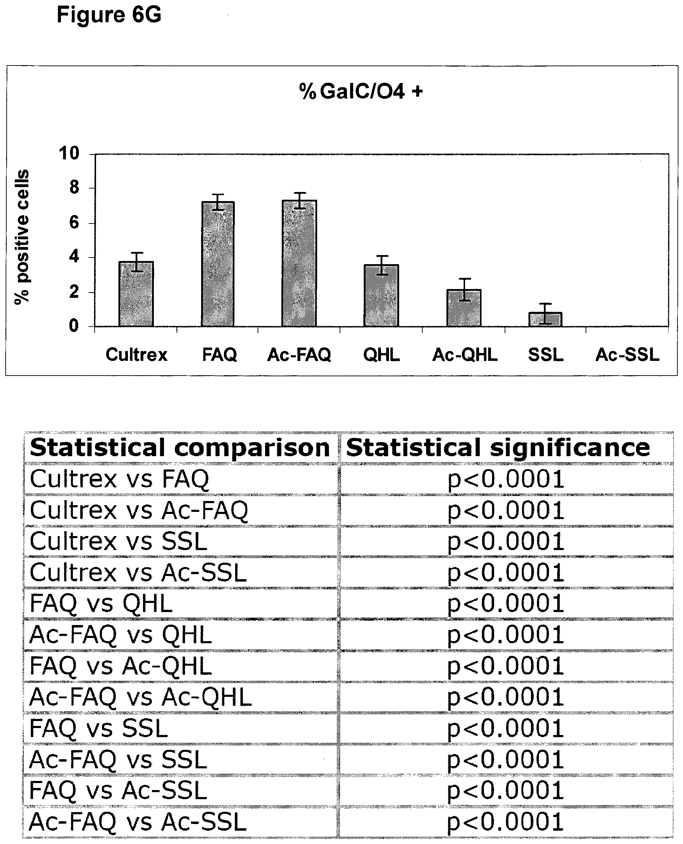

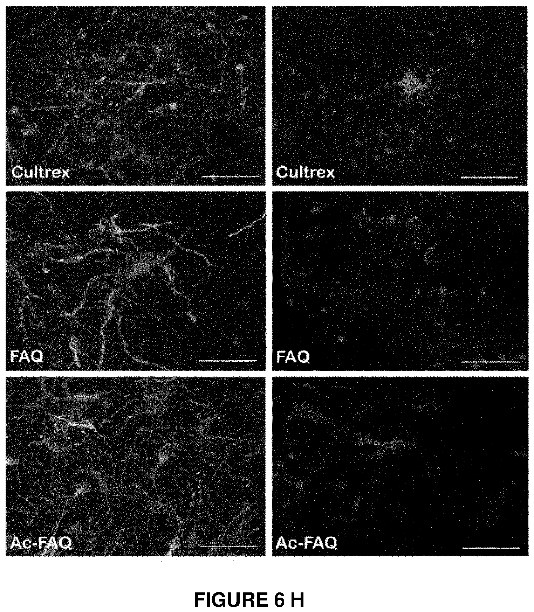

Differentiation of both murine and human NSCs, showed similar results (figure 6).

NSCs cultured on bare plastic wells did not show an appreciable branching, neither an efficacious differentiation thus the clustered NSC progeny could not be reliably quantified in terms of differentiated phenotypes.

The results show that the SAPs according to the present invention allow NSC differentiation into neurons, astrocytes and oligodendrocytes. The number of neurons and oligodendrocytes obtained by differentiating the cells on FAQ or Ac-FAQ were significantly higher even when compared to the progenies obtained on Cultrex (figure 6A-6C-6E-6G), a positive control. Oligodendrocytes were detected in the shape of discrete clusters of GalC/O4 positive cells on Cultrex, FAQ and Ac-FAQ (figure 6D-6H).

These results show that both FAQ and Ac-FAQ efficiently support human and murine NSCs proliferation.

βIIITubulin positive neurons (murine, left column of D; human, left column of H), GFAP positive astrocytes (murine, left column of D; human, left column of H) and GalC/O4 double positive oligodendrocytes (murine, right column of D; human, right column of H) are detected in both the tested materials. Nuclei are visualized with DAPI. Scale bars = 50 $\mu$m.

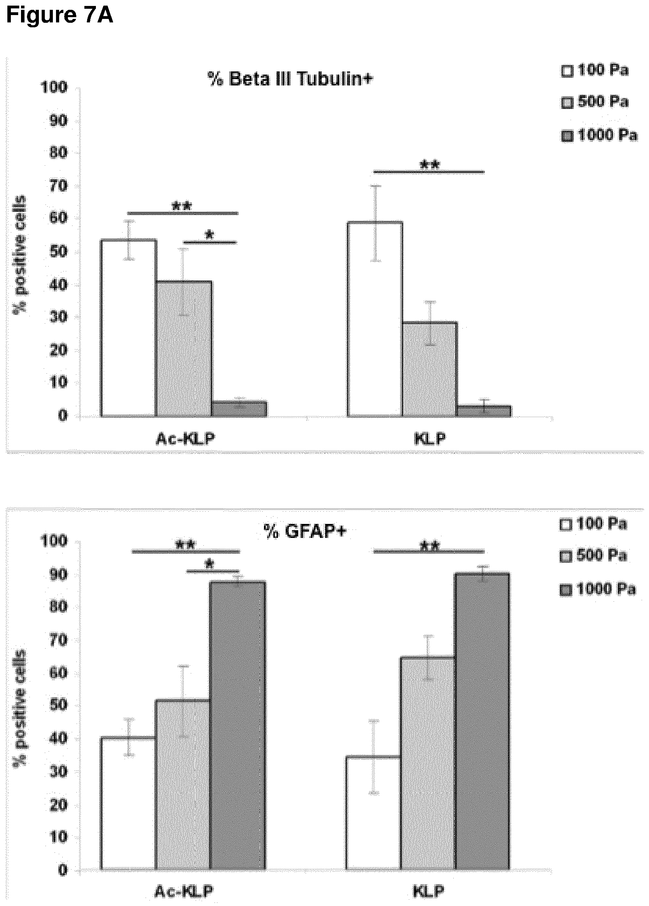

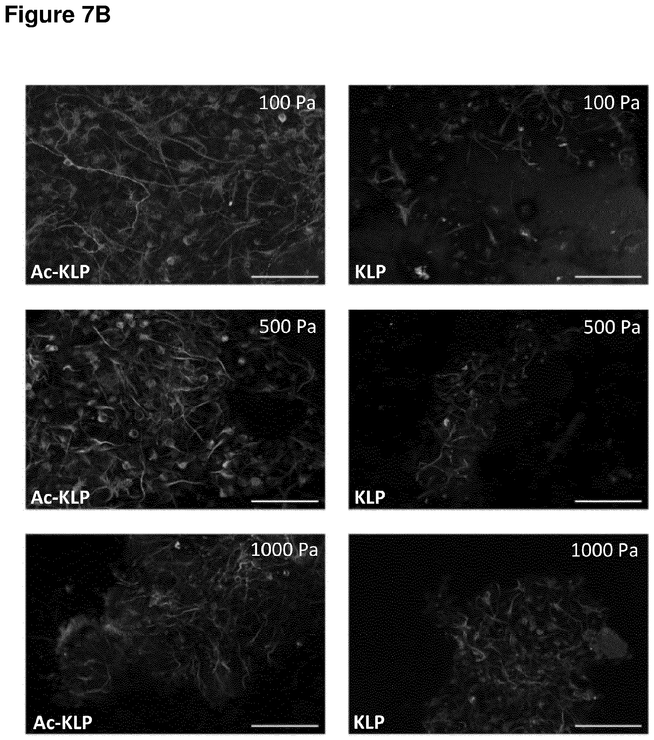

For both KLP and Ac-KLP the tested different concentrations showed significant increments of differentiated neurons (βIIITubulin positive cells) in correspondence of stiffness reductions (Figure 7A): this was counterbalanced by increments in the percentage of astrocytes (GFAP positive cells). (*=P<0.05 **=P<0.01). No significant difference was detected between KLP and Ac-KLP at similar scaffold stiffnesses. Remarkably, percentages of neurons above 50% are the best results achieved among all the tested functionalized scaffolds. For softer scaffolds, neuronal and astroglial morphologies were highly branched and spread all over the scaffolds (Figure 7B).

**Example 9.**

*In vivo* regeneration of spinal cord injuries

Surgery

The results of the neuroregenerative potential of Ac-FAQ SAPeptides in an *in vivo* model of acute contusive spinal cord injury (SCI) are described below.

All procedures involving animals were performed according to EC guidelines (EC Council Directive 86/609, 1987).

The results of the neuroregenerative potential of Ac-FAQ SAPeptides in an *in vivo* model of acute contusive spinal cord injury (SCI) are described herein.

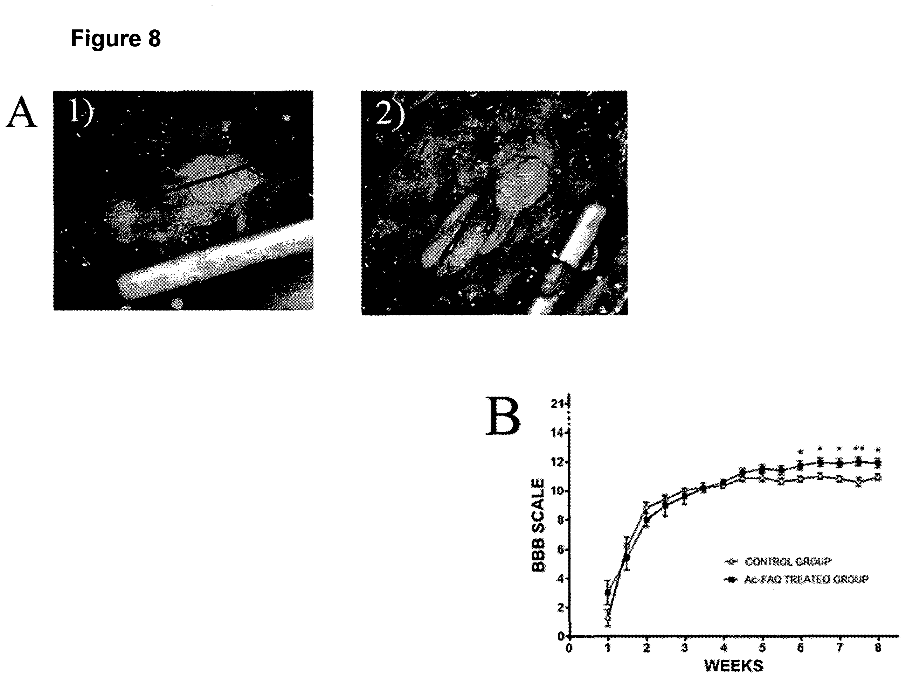

20 female Sprague-Dawley rats weighting 200-250 gr (Charles River Laboratories) were randomly divided into two groups: 1) spinal cord injured animals treated with Ac-FAQ and 2) spinal cord injured animals treated with the saline solution (control group). Rats were anesthetized with an intraperitoneal injection of ketamine (80 mg/kg) and xylazine (10 mg/kg). After laminectomy at the T9-T10 level, the exposed spinal cord was lesioned (Figure 8A1) by a 10-gram rod dropped from a height of 25 mm by using a MASCIS Impactor device (WM Keck Center for Collaborative Neuroscience, Rutgers University), as previously described 33. Immediately after injury, animals were injected with Ac-FAQ (1% aqueous solution) or the saline solution using an Hamilton syringe held via a micromanipulator (Figure 8A2). The biomaterial or the distilled water was delivered into the lesion site at distances of 500 $\mu$m, and at each interval two injections of 0,5 $\mu$l each were made, for a total dose of 3 $\mu$l. After injection, the muscles were sutured and the skin was closed with wound clips. Rats were treated daily for one week with analgesic (carprofen, 5 mg/kg) and antibiotic (enrofloxacin, 5 mg/kg). Bladder was manually expressed until recovery of the bladder reflex.

Behavioural test

Hindlimb recovery was assessed by using the Basso, Beattie, Bresnahan (BBB) Locomotor Rating Scale. Starting from 1 week post-injury, every 3-4 days each rat was observed and recorded with a digital video camera for 4 min in an open field. Rats were evaluated in double-blind experimental settings. Ac-FAQ treated animals showed a gradual recovery and a significant improvement (p<0.05) of hindlimb motor recovery in comparison to controls (Figure 8B) at week 6,7 and 8 weeks after injury (11.95 ± 0.30): showing a frequent or consistent plantar stepping with occasional forelimbs-hindlimbs coordination. BBB score of the vehicle-treated animals plateaued at week 5 (10.92 ± 0.27). Control group's BBB scores are consistent with the fact that injections did not further impair the locomotor functions of the injured animals. Ac-FAQ treated animals showed improvement of both hindlimbs motor function and coordination and a better recovery of plantar stepping and coordination in comparison to controls.

## Example 10.

In vivo regeneration of spinal cord injuries

Histology and morphometrical quantification

8 weeks after injury animals were sacrificed by transcardial perfusion with 4% paraformaldehyde. At this time the lesion site is occupied by a cyst, that can be spanned by strands of connective tissue (trabeculae) occasionally containing axon sprouts. Spinal cords were removed, embedded in OCT, frozen and sliced into 16 $\mu$m thick longitudinal sections.

For histochemical analysis, slices were stained with hematoxylin/eosin. For immunofluorescence analysis, slices were washed with PBS, permeabilized with 0,1 % Triton X-100 and treated with 10% normal goat serum.

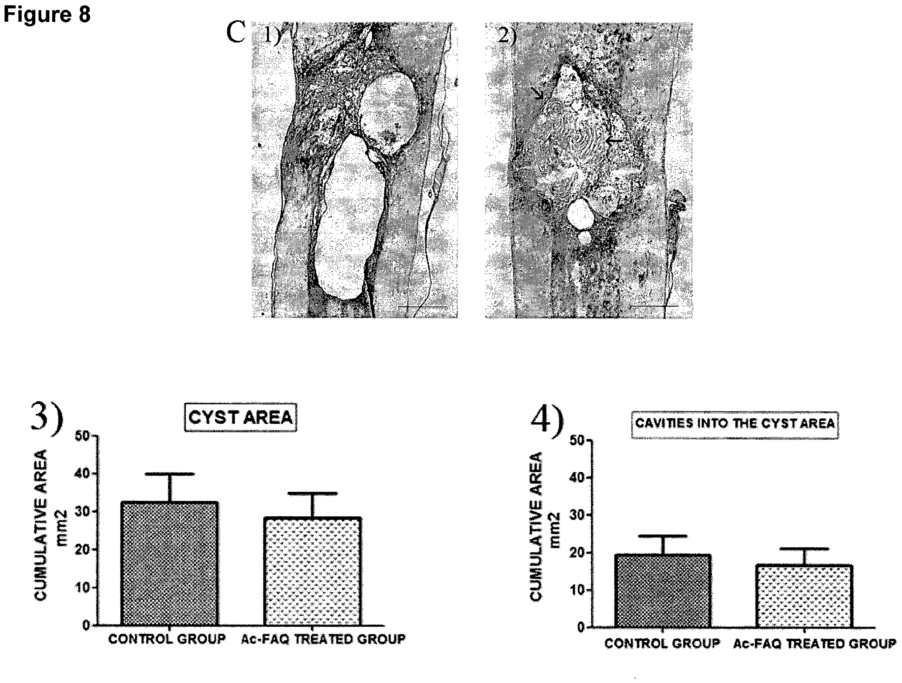

Quantitative analysis was performed on longitudinal sections of rat spinal cords, spanning between T7 and T12 and including the injury site (T9-T10). Average cumulative cyst area in Ac-FAQ-treated animals (28.30 ± 6.70 mm$^2$) was similar to that one of controls (32.30 ± 7.69mm$^2$), suggesting that injection of the Ac-FAQ did not significantly affect cyst formation. Hence, in order to assess if the biomaterial injection could favour the projection of trabeculae into the cyst and contribute to bypass the scar, we measured only cavities into the cyst excluding trabeculae. However, measurements of the cavities size within the cyst, obtained by excluding strands of trabeculae did not show any significant difference between Ac-FAQ and control groups (16.77 ± 4.50 mm$^2$ and 19.46 ± 4.97 mm$^2$, respectively) (Figure 8C).

The presence of axons expressing Gap-43 and $\beta$III within the cyst was investigated. Gap-43 positive fibers were quantified and the relative value of GAP-43 immunopositive area was expressed as percentage of the total cyst area.

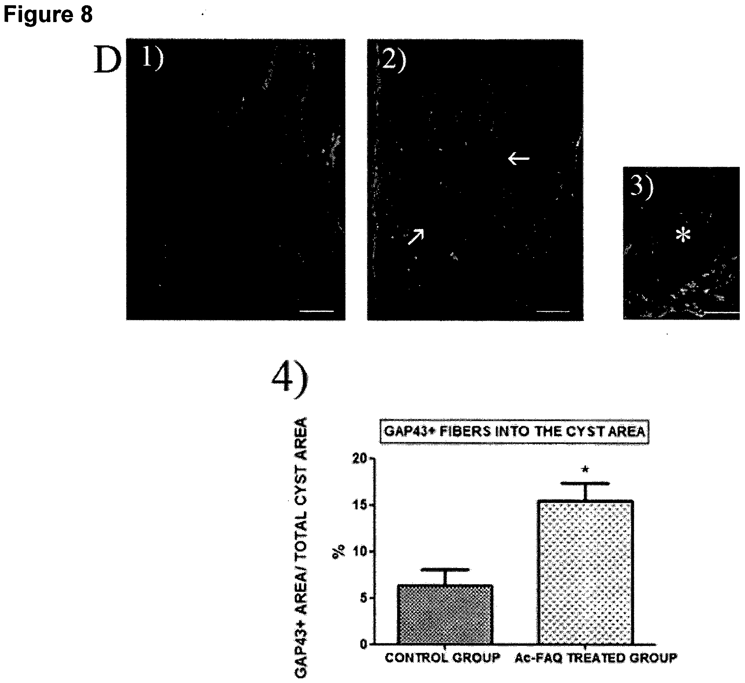

The presence of higher levels of GAP-43 in the lesion site of SAP-injected in comparison to saline-injected animals suggests that SAP provides microenvironments promoting neurite outgrowth mediated by GAP-43 and allows supplementary axonal sprouting, thus fostering nervous regeneration. The average immunopositivity for GAP-43 was significantly higher in animals treated with SAPs (15.46 ± 1.94 % of the whole cyst area) than in controls (6.33 ± 1.70 % of the whole cyst area) (Figure 8D). Figure 5D2-5D3 show SAP-treated animals where we observed several GAP43 positive fibers (white arrows) infiltrating the cyst (D3, high magnification of D2; *= cyst) while figure 8D1 depict the cyst of a saline-injected animal. The relative value of GAP-43 positive area, expressed as percentage of the total cyst area, was significantly higher in SAP-treated than in vehicle-injected animals (D4): p<0.05. Scale bar: (D1) and (D2) = 400 $\mu$m, (D3) = 200 $\mu$m. Nuclei were counterstained with DAPI.

Injured tissue was quantified in seven-to-sixteen longitudinal sections per animal, depending on the cyst extent. Sections started from the dorsal surface of the spinal cord and were spaced 160 $\mu$m. Sections were stained with hematoxylin/eosin and images were captured (Axioplan2 Zeiss microscope) at 5x magnification, then single images were merged, converted into binary pictures and cavities into the injured area were quantified via Image J software (http://rsb.info.nih.gov/ij/). We measured also the whole injured tissue size. Pixel area was converted to mm$^2$ and measurements either of the injured tissue or of the cavities inside it were added to produce the cumulative area of each animal as relative measure of the lesion size.

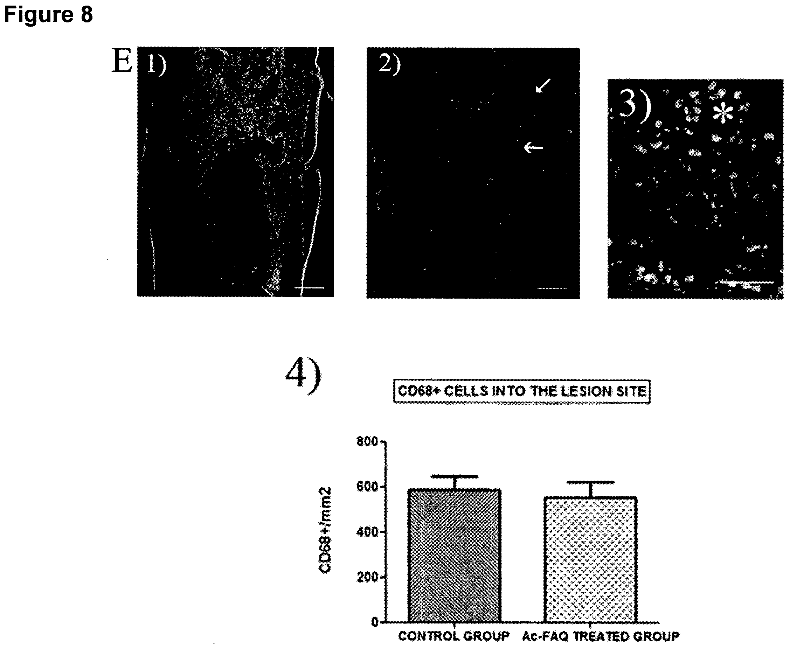

Macrophage infiltration, into the lesion site, was quantified in three longitudinal sections, spaced 160 $\mu$m, per animal. Images (20x magnification) were captured of immunostained tissues with CD68 (1:500, Serotec) and an Alexa 488-conjugated antibodies (1:500, Invitrogen). A total area of 2.3 mm$^2$ was quantified per each section (approximately 16 images). Cells count of CD68 positive cells was made with Image J. The number of macrophages/mm$^2$ was averaged over three measurements on different sections per each animal (Figure 8E).

Axon regeneration/sprouting, into the cyst area, was quantified on six longitudinal sections (spaced 160 $\mu$m) per animal via GAP-43 (1:200, Millipore) and Cy3-conjugated antibodies (1:1000, Jackson). For each section we took serial images (10X magnification) of the injured site. Again, images were merged, converted to binary pictures and the content of positive pixels was measured in order to quantify the GAP-43 positive area into the injured site. The mean of the six measurements, expressed as percentage of the total cyst area, constituted the value of the axonal regeneration/sprouting of each animal.

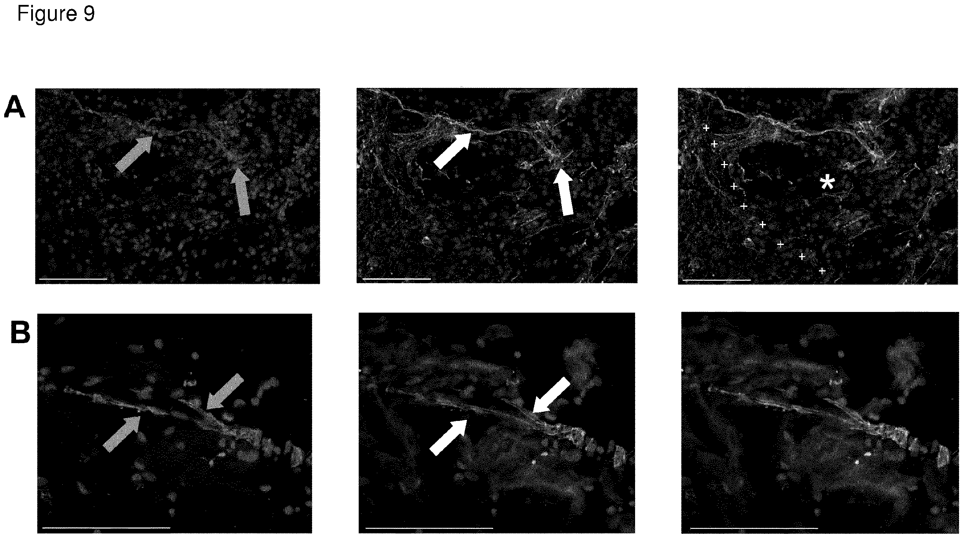

In Ac-FAQ-treated animals we detected several fibers invading both the cyst cavity and trabeculae, whereas in saline-injected animals the majority of fibers was only observed into trabeculae, indicating that the biomaterial could also constitute a trophic support for growing axons. All GAP-43 positive fibers also expressed βIII (Figure 9A). Gap-43 is a phosphoprotein that is known to play an important role in neural development, axonal regeneration, and modulation of synaptic function.

To stain proteoglycans and glycosaminoglycans into the lesion site we used toluidine blue O solution (Sigma), while Collagen content was evaluated using trichrome Heidenhein modified method kit (Bio-Optica).Other primary antibodies comprise anti-laminin (1:200, Sigma), anti-Iba1 (1:1000, Wako) for microglia and anti-GFAP (1:500, Millipore) for astrocytes.

Further histological analyses were performed and it could be seen that several cells and nerve fibers infiltrated the SAP scaffolds. These cells were positive for Iba1, a marker for microglia, for βIIITubulin, a marker for neurons, and GFAP (glial fibrillary acidic protein), a marker for astrocytes. Infiltrating nerve fibers were positive for both GAP-43 and βIIITubulin (Figure 9B).

Taken together, data from histology suggest that Ac-FAQ could promote a further axonal sprouting/regeneration in comparison to the limited axon outgrowth normally occurring after incomplete SCI. This finding is in accordance with the improvement of locomotor recovery that we observed in biomaterial-treated animals.

Statistical analysis

Data were processed using GraphPadPrism 5 software. Values are reported as means ± standard error of the mean (SEM). Results of NSC proliferation assays and mNSC differentiation in the presence of the soluble KLPGWSG peptide were assessed for their statistical significance via paired t-tests: results were compared to negative control. Statistical significance of scaffold-based differentiation tests was assessed using the one-way ANOVA analysis followed by Tukey's comparison test. In all tests p values < 0.05 were considered as statistically significant.

BBB score significance test between control and treated animal groups was performed by unpaired t-test. For quantification of the cavity size, macrophage infiltration and GAP43 positive fibers, groups comparison was made by Mann-Whitney test. All analyses were two-tailed and p values < 0.05 were considered as statistically significant.

From the above description and the above-noted examples, the advantage attained by the product described and obtained according to the present invention are apparent.

**References**

1. Polak, J. M.; Bishop, A. E., Stem Cells and Tissue Engineering: Past, Present, and Future. Ann N Y Acad Sci 2006,1068, 352-66.

2. Weiss, S.; Reynolds, B. A.; Vescovi, A. L.; Morshead, C.; Craig, C. G.; van der Kooy, D., Is There a Neural Stem Cell in the Mammalian Forebrain? Trends Neurosci 1996,19, 387-93.

3. Kang, H. C.; Kim, D. S.; Kim, J. Y.; Kim, H. S.; Lim, B. Y.; Kim, H. D.; Lee, J. S.; Eun, B. L.; Kim, D. W., Behavioral Improvement after Transplantation of Neural Precursors Derived from Embryonic Stem Cells into the Globally Ischemic Brain of Adolescent Rats. Brain Dev 2009.

4. Zhang, P.; Li, J.; Liu, Y.; Chen, X.; Kang, Q.; Zhao, J.; Li, W., Human Neural Stem Cell Transplantation Attenuates Apoptosis and Improves Neurological Functions after Cerebral Ischemia in Rats. Acta Anaesthesiol Scand 2009,53, 1184-91.

5. Pluchino, S.; Quattrini, A.; Brambilla, E.; Gritti, A.; Salani, G.; Dina, G.; Galli, R.; Del Carro, U.; Amadio, S.; Bergami, A., et al., Injection of Adult Neurospheres Induces Recovery in a Chronic Model of Multiple Sclerosis. Nature 2003,422, 688-94.

6. Vescovi, A. L.; Reynolds, B. A.; Fraser, D. D.; Weiss, S., Bfgf Regulates the Proliferative Fate of Unipotent (Neuronal) and Bipotent (Neuronal/Astroglial).

7. Taraballi, F; Campione M, Vescovi AL, Sassella A, Paleari A, Whang W, Gelain F Effect of Functionalization on the Self-Assembling Propensity of β-Sheet Forming Peptides. Soft Matter 5: 660-668.

8. Gelain, F.; Bottai, D.; Vescovi, A.; Zhang, S., Designer Self-Assembling Peptide Nanofiber Scaffolds for Adult Mouse Neural Stem Cell 3-Dimensional Cultures. PLoS ONE 2006,1, e119.

**Claims**

1. A self-assembling peptide (SAP) having the sequence:
   X-FAQRVPPGGGLDLKLDLKLDLK- Y;
   wherein X is $NH_2$ or Ac, and wherein Y is $CONH_2$ or COOH.

2. The self-assembling peptide according to claim 1, of sequence:
   $NH_2$-FAQRVPPGGGLDLKLDLKLDLK-$CONH_2$ SEQ ID N.16.

**3.** The self-assembling peptide according to claim 1, of sequence:
Ac-FAQRVPPGGGLDLKLDLKLDLK-CONH$_2$ SEQ ID N.17.

**4.** The self-assembling peptide according to claim 1, of sequence:
NH$_2$-FAQRVPPGGGLDLKLDLKLDLK-COOH SEQ ID NO. 46.

**5.** The self-assembling peptide according to claim 1, of sequence:
Ac-FAQRVPPGGGLDLKLDLKLDLK-COOH SEQ ID NO. 47.

**6.** A hydrogel comprising the self-assembling peptides according to anyone of claims 1 to 5 and a hydrogelating ingredient.

**7.** A self-assembling peptide polymer comprising at least two self-assembling peptides according to anyone of claims 1 to 5.

**8.** A tabular nanofiber comprising at least two self-assembling peptides according to anyone of claims 1 to 5.

**9.** A complex interwoven membrane comprising at least two tabular nanofibres according to claim 8.

**10.** A self-assembling peptide polymer comprising at least two self-assembling peptides according to anyone of claims 1 to 5, for use as a medicament.

**11.** A self-assembling peptide polymer comprising at least two self-assembling peptides according to anyone of claims 1 to 5, for use as a scaffold in tissue regeneration, wherein said tissue is preferably a tissue of the central nervous system.

**12.** A self-assembling peptide according to anyone of claims 1 to 5, for use as a biomaterial and as a cell culture substrate.

**Patentansprüche**

**1.** Selbstassemblierendes Peptid (SAP), das die folgende Sequenz aufweist: X-FAQRVPPGGGLDLKLDLKLDLK-Y; wobei X NH$_2$ oder Ac ist und wobei Y CONH$_2$ oder COOH ist.

**2.** Selbstassemblierendes Peptid nach Anspruch 1 mit der Sequenz:
NH$_2$-FAQRVPPGGGLDLKLDLKLDLK-CONH$_2$ SEQ ID N.16.

**3.** Selbstassemblierendes Peptid nach Anspruch 1 mit der Sequenz:
Ac-FAQRVPPGGGLDLKLDLKLDLK-CONH$_2$ SEQ ID N.17.

**4.** Selbstassemblierendes Peptid nach Anspruch 1 mit der Sequenz:
NH$_2$-FAQRVPPGGGLDLKLDLKLDLK-COOH SEQ ID NO. 46.

**5.** Selbstassemblierendes Peptid nach Anspruch 1 mit der Sequenz:
Ac-FAQRVPPGGGLDLKLDLKLDLK-COOH SEQ ID NO. 47.

**6.** Hydrogel, umfassend die selbstassemblierenden Peptide nach einem der Ansprüche 1 bis 5 und einen Hydrogel-bildenden Inhaltsstoff.

**7.** Selbstassemblierendes Peptidpolymer, umfassend mindestens zwei selbstassemblierende Peptide nach einem der Ansprüche 1 bis 5.

**8.** Flache Nanofaser, umfassend mindestens zwei selbstassemblierende Peptide nach einem der Ansprüche 1 bis 5.

**9.** Komplex verwobene Membran, umfassend mindestens zwei flache Nanofasern nach Anspruch 8.

**10.** Selbstassemblierendes Peptidpolymer, umfassend mindestens zwei selbstassemblierende Peptide nach einem der Ansprüche 1 bis 5, zur Verwendung als Medikament.

11. Selbstassemblierendes Peptidpolymer, umfassend mindestens zwei selbstassemblierende Peptide nach einem der Ansprüche 1 bis 5, zur Verwendung als Gerüst bei der Geweberegeneration, wobei das Gewebe vorzugsweise ein Gewebe des Zentralnervensystems ist.

12. Selbstassemblierendes Peptid nach einem der Ansprüche 1 bis 5 zur Verwendung als Biomaterial und als Zellkultursubstrat.

**Revendications**

1. Un peptide à auto-assemblage (SAP) ayant la séquence :
X-FAQRVPPGGGLDLKLDLKLDLK-Y ;
dans lequel X est $NH_2$ ou Ac, et dans lequel Y est $CONH_2$ ou COOH.

2. Le peptide à auto-assemblage selon la revendication 1, de séquence :
$NH_2$-FAQRVPPGGGLDLKLDLKLDLK-$CONH_2$ SEQ ID N.16.

3. Le peptide à auto-assemblage selon la revendication 1, de séquence :
Ac-FAQRVPPGGGLDLKLDLKLDLK-$CONH_2$ SEQ ID N.17.

4. Le peptide à auto-assemblage selon la revendication 1, de séquence :
$NH_2$-FAQRVPPGGGLDLKLDLKLDLK-COOH SEQ ID NO. 46.

5. Le peptide à auto-assemblage selon la revendication 1, de séquence :
Ac-FAQRVPPGGGLDLKLDLKLDLK-COOH SEQ ID NO. 47.

6. Un hydrogel comprenant les peptides à auto-assemblage selon l'une quelconque des revendications 1 à 5 et un ingrédient d'hydrogélation.

7. Un polymère de peptides à auto-assemblage comprenant au moins deux peptides à auto-assemblage selon l'une quelconque des revendications 1 à 5.

8. Une nanofibre tabulaire comprenant au moins deux peptides à auto-assemblage selon l'une quelconque des revendications 1 à 5.

9. Une membrane entrelacée complexe comprenant au moins deux nanofibres tabulaires selon la revendication 8.

10. Un polymère de peptides à auto-assemblage comprenant au moins deux peptides à auto-assemblage selon l'une quelconque des revendications 1 à 5, pour une utilisation comme médicament.

11. Un polymère de peptides à auto-assemblage comprenant au moins deux peptides à auto-assemblage selon l'une quelconque des revendications 1 à 5, pour une utilisation comme échafaudage dans une régénération tissulaire, dans lequel ledit tissu est de préférence un tissu du système nerveux central.

12. Un peptide à auto-assemblage selon l'une quelconque des revendications 1 à 5, pour une utilisation comme biomatériau et comme substrat de culture de cellules.

**Figure 1B**

Beta III Tubulin staining

Cultrex — #HAI, #GET, #KLP, Untreated

Laminin — #HAI, #GET, #KLP, Untreated

Figure 2

# Figure 3A

Frequency Sweep

Figure 3B

Figure 4

EP 2 686 338 B1

# Figure 5

**Figure 5 C**

Cell Viability Assay

mNSCs

EP 2 686 338 B1

**Figure 5 D**

Cell Viability Assay

## Figure 6A

| Statistical comparison | Statistical significance |
|---|---|
| Cultrex vs FAQ | $p < 0.0001$ |
| Cultrex vs Ac-FAQ | $p < 0.0001$ |
| Cultrex vs QHL | $p < 0.0001$ |
| Cultrex vs Ac-QHL | $p < 0.0001$ |
| Cultrex vs SSL | $p < 0.0001$ |
| Cultrex vs Ac-SSL | $p < 0.0001$ |
| FAQ vs QHL | $p < 0.0001$ |
| FAQ vs Ac-QHL | $p < 0.0001$ |
| Ac-FAQ vs QHL | $p < 0.0001$ |
| Ac-FAQ vs Ac-QHL | $p < 0.0001$ |
| FAQ vs SSL | $p < 0.0001$ |
| Ac-FAQ vs SSL | $p < 0.0001$ |
| FAQ vs Ac-SSL | $p < 0.0001$ |
| Ac-FAQ vs Ac-SSL | $p < 0.0001$ |

## Figure 6B

| Statistical comparison | Statistical significance |
|---|---|
| Cultrex vs FAQ | p<0.0001 |
| Cultrex vs Ac-FAQ | p<0.0001 |
| Cultrex vs Ac-QHL | p<0.05 |
| Cultrex vs SSL | p<0.005 |
| Cultrex vs Ac-SSL | p<0.0001 |
| FAQ vs QHL | p<0.0001 |
| FAQ vs Ac-QHL | p<0.0001 |
| FAQ vs SSL | p<0.0001 |
| FAQ vs Ac-SSL | p<0.0001 |
| Ac-FAQ vs QHL | p<0.0001 |
| Ac-FAQ vs Ac-QHL | p<0.0001 |
| Ac-FAQ vs SSL | p<0.0001 |
| Ac-FAQ vs Ac-SSL | p<0.0001 |

## Figure 6C

| Statistical comparison | Statistical significance |
|---|---|
| Cultrex vs FAQ | p<0.0001 |
| Cultrex vs Ac-FAQ | p<0.0001 |
| Cultrex vs Ac-QHL | p<0.005 |
| Cultrex vs SSL | p<0.05 |
| Cultrex vs Ac-SSL | p<0.0001 |
| FAQ vs Ac-FAQ | p<0.005 |

# Figure 6

## Figure 6E

| Statistical comparison | Statistical significance |
|---|---|
| Cultrex vs FAQ | p<0.0001 |
| Cultrex vs Ac-FAQ | p<0.0001 |
| Cultrex vs SSL | p<0.0001 |
| Cultrex vs Ac-SSL | p<0.0001 |
| FAQ vs QHL | p<0.0001 |
| Ac-FAQ vs QHL | p<0.005 |
| FAQ vs Ac-QHL | p<0.005 |
| FAQ vs SSL | p<0.0001 |
| Ac-FAQ vs SSL | p<0.0001 |
| FAQ vs Ac-SSL | p<0.0001 |
| Ac-FAQ vs Ac-SSL | p<0.0001 |

## Figure 6F

| Statistical comparison | Statistical significance |
|---|---|
| Cultrex vs FAQ | p<0.05 |
| Cultrex vs SSL | p<0.05 |
| Cultrex vs Ac-SSL | p<0.0001 |
| FAQ vs QHL | p<0.05 |
| Ac-FAQ vs QHL | p<0.05 |
| FAQ vs Ac-QHL | p<0.05 |
| FAQ vs SSL | p<0.05 |
| Ac-FAQ vs SSL | p<0.05 |
| FAQ vs Ac-SSL | p<0.0001 |
| Ac-FAQ vs Ac-SSL | p<0.005 |

## Figure 6G

| Statistical comparison | Statistical significance |
| --- | --- |
| Cultrex vs FAQ | p<0.0001 |
| Cultrex vs Ac-FAQ | p<0.0001 |
| Cultrex vs SSL | p<0.0001 |
| Cultrex vs Ac-SSL | p<0.0001 |
| FAQ vs QHL | p<0.0001 |
| Ac-FAQ vs QHL | p<0.0001 |
| FAQ vs Ac-QHL | p<0.0001 |
| Ac-FAQ vs Ac-QHL | p<0.0001 |
| FAQ vs SSL | p<0.0001 |
| Ac-FAQ vs SSL | p<0.0001 |
| FAQ vs Ac-SSL | p<0.0001 |
| Ac-FAQ vs Ac-SSL | p<0.0001 |

**FIGURE 6 H**

# Figure 7A

# Figure 7B

**Figure 8**

**Figure 8**

EP 2 686 338 B1

Figure 8

Figure 8

Figure 9

EP 2 686 338 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2011053838 W

**Non-patent literature cited in the description**

- Egf-Generated Cns Progenitor Cells. *Neuron,* 1993, vol. 11 (9), 51-66
- **POLAK, J. M. ; BISHOP, A. E.** Stem Cells and Tissue Engineering: Past, Present, and Future. *Ann N Y Acad Sci,* 2006, vol. 1068, 352-66
- **WEISS, S. ; REYNOLDS, B. A. ; VESCOVI, A. L. ; MORSHEAD, C. ; CRAIG, C. G. ; VAN DER KOOY, D.** Neural Stem Cell in the Mammalian Forebrain?. *Trends Neurosci,* 1996, vol. 19, 387-93
- **KANG, H. C. ; KIM, D. S. ; KIM, J. Y. ; KIM, H. S. ; LIM, B. Y. ; KIM, H. D. ; LEE, J. S. ; EUN, B. L. ; KIM, D. W.** Behavioral Improvement after Transplantation of Neural Precursors Derived from Embryonic Stem Cells into the Globally Ischemic Brain of Adolescent Rats. *Brain Dev,* 2009
- **ZHANG, P. ; LI, J. ; LIU, Y. ; CHEN, X. ; KANG, Q. ; ZHAO, J. ; LI, W.** Human Neural Stem Cell Transplantation Attenuates Apoptosis and Improves Neurological Functions after Cerebral Ischemia in Rats. *Acta Anaesthesiol Scand,* 2009, vol. 53, 1184-91
- **PLUCHINO, S. ; QUATTRINI, A. ; BRAMBILLA, E. ; GRITTI, A. ; SALANI, G. ; DINA, G ; GALLI, R. ; DEL CARRO, U. ; AMADIO, S. ; BERGAMI, A. et al.** Injection of Adult Neurospheres Induces Recovery in a Chronic Model of Multiple Sclerosis. *Nature,* 2003, vol. 422, 688-94
- **VESCOVI, A. L. ; REYNOLDS, B. A. ; FRASER, D. D. ; WEISS, S.** *Bfgf Regulates the Proliferative Fate of Unipotent (Neuronal) and Bipotent (Neuronal/Astroglial*
- **TARABALLI, F ; CAMPIONE M ; VESCOVI AL ; SASSELLA A ; PALEARI A ; WHANG W ; GELAIN F.** Effect of Functionalization on the Self-Assembling Propensity of β-Sheet Forming Peptides. *Soft Matter,* vol. 5, 660-668
- **GELAIN, F. ; BOTTAI, D. ; VESCOVI, A. ; ZHANG, S.** Designer Self-Assembling Peptide Nanofiber Scaffolds for Adult Mouse Neural Stem Cell 3-Dimensional Cultures. *PLoS ONE,* 2006, vol. 1, e119